# EUROPÄISCHE PATENTSCHRIFT

(11) **EP 1 774 988 B1**
(45) Veröffentlichungstag und Bekanntmachung des Hinweises auf die Patenterteilung: **19.05.2010**
(21) Anmeldenummer: 06018400.9
(22) Anmeldetag: 02.09.2006
(51) Int. Cl.: A61N 1/37, A61B 5/04, A61N 1/39, A61N 1/362

(54) **Implantierbare Vorrichtung zur Herzvektor-Bestimmung**
Implantable cardiac vector determination device
Dispositif implantable pour la détermination des vecteurs cardiaques

(30) Priorität: 11.10.2005 DE 102005049009
(43) Veröffentlichungstag der Anmeldung: 18.04.2007
(73) Patentinhaber: Biotronik CRM Patent AG, 6341 Baar (CH)
(72) Erfinder: Dörr, Thomas, 12437 Berlin (DE); Kucher, Andreas, Dr., 16303 Schwedt (DE); Philipp, Jens, Dr., 10555 Berlin (DE); Weiss, Ingo, Dr., 12435 Berlin (DE)
(74) Vertreter: Lindner-Vogt, Karin L.

(56) Entgegenhaltungen:
- WO-A-00/57781
- US-A1- 2005 192 505

## Beschreibung

Die Erfindung betrifft ein implantierbares Elektrostimulationsgerät für ein Herz, insbesondere einen implantierbaren Kardioverter/Defibrillator.

Implantierbare Herzschrittmacher und implantierbare Kardioverter/Defibrillatoren haben einen hohen Entwicklungsstand erreicht. Die Geräte müssen über Jahre hinweg zuverlässig arbeiten, da sie im implantierten Zustand nur auf dem Wege über eine Operation zugänglich sind. Dies setzt der Komplexität der Geräte Grenzen. Andererseits sollen Herzschrittmacher und Defibrillatoren in der Lage sein, pathologische Zustände des zu behandelnden Herzens sowohl mit hoher Sensitivität als auch mit hoher Spezifität zu erfassen, damit einerseits ein behandlungsbedürftiger Zustand nicht unerkannt bleibt und andererseits eine unnötige oder falsche Behandlung möglichst vermieden wird. Im Zusammenhang mit implantierbaren Kardiovertern/Defibrillatoren (ICDs) sei hier die Unterscheidung zwischen Tachykardien, die ihren Ursprung im Ventrikel haben (ventrikuläre Tachykardien), und supraventrikulären Tachykardien genannt, die ihren Ursprung beispielsweise im Atrium haben.

Aus dem Dokument US 2005/192505 ist eine Vorrichtung nach dem Oberbegriff des Anspruchs 1 bekannt.

Weiterhin besteht nach wie vor das Problem, einen Therapieerfolg möglichst zuverlässig zu erkennen um andernfalls die Therapie anpassen zu können. Im einfachsten Fall betrifft dies das Einstellen der Stärke eines Stimulationsimpulses. Dieser soll gerade so bemessen sein, dass das Herzgewebe (Myocard) der stimulierten Herzkammer - also Vorhof (Atrium) oder Ventrikel - auf den Stimulationsimpuls anspricht und in der Folge kontrahiert. Auf der anderen Seite sollen zu starke Stimulationsimpulse vermieden werden, um einen unnötigen Energieverbrauch zu vermeiden, denn die Energie muss einer Batterie entnommen werden, deren Erschöpfung eine Operation und einen Austausch des Stimulationsgerätes notwendig macht. Gerade bei biventrikulären Schrittmachern für die Resynchronisationstherapie (CRT: cardiac resynchronization therapy) macht die Erfassung des Stimulationserfolges (capture detection) insbesondere im linken Ventrikel nach wie vor Probleme.

Zur Lösung der hier angedeuteten Probleme sind eine Reihe von Einzellösungen bekannt.

Ziel der vorliegenden Erfindung ist es, ein Herzstimulationsgerät zu schaffen, welches über Mittel verfügt, die die bekannten Mittel zur Lösung der angedeuteten Probleme sinnvoll ergänzen oder gar ersetzen können.

Erfindungsgemäß wird dieses Ziel mit einem implantierbaren Elektrostimulationsgerät mit wenigstens drei Elektrodenleitungen, welche jeweils mindestens eine Elektrode aufweisen oder mit Anschlüssen zur Verbindung mit wenigstens drei Elektrodenleitungen, welche jeweils mindestens eine Elektrode aufweisen, erreicht, welches wenigstens drei Eingangskanäle aufweist, die mit wenigstens einer solchen Elektrode oder mit einem Anschluss für eine solche Elektrode verbunden sind, mit der sich wenigstens drei verschiedene, mit einer Erregung des Herzgewebes (Myokard) einhergehende elektrische Potentiale im Herzen erfassen lassen. Jeder der Eingangskanäle bildet einen Sensing-Kanal. Das Elektrostimulationsgerät weist eine Signalverarbeitungseinheit auf, die mit den drei Eingangs- bzw. Sense-Kanälen verbunden ist und die ausgebildet ist, den zeitlichen Verlauf der über die drei Sense-Kanäle erfassten Potentiale als drei Eingangssignale in zeitlichem Bezug zu einem periodisch wiederkehrenden Triggersignal auszuwerten. Das Triggersignal löst ein Zeitfenster aus. Die Signalverarbeitungseinheit ist weiterhin ausgebildet, für jedes der drei Eingangssignale vorgegebene Signalmerkmale innerhalb des von dem Triggersignal ausgelösten Zeitfensters zu erfassen, zu speichern und mit entsprechenden Signalmerkmalen vorangegangener Zeitfenster desselben Sense-Kanals zu vergleichen. Die Signalverarbeitungseinheit ist weiterhin ausgebildet, einander entsprechende Signalmerkmale der von verschiedenen Sense-Kanälen stammenden Eingangssignale innerhalb desselben Zeitfensters miteinander zu vergleichen.

Die Eingangs- bzw. Sense-Kanäle können dabei mit einer intrakardial anzuordnenden Elektrode verbunden sein oder verbunden werden sowie einer Neutralelektrode, beispielsweise einem Schrittmachergehäuse, als Gegenelektrode. Die von den Sense-Kanälen erfassten Signale sind dann unipolare Signale.

Bevorzugt ist es jedoch, wenn jeder der Sense-Kanäle mit zwei im Herzen oder in der Peripherie des Herzens angeordneten Elektroden verbunden ist, so dass jeder der Sense-Kanäle ein bipolares Eingangssignal erfasst. Für drei bipolare Sense-Kanäle werden mindestens drei Elektroden benötigt, von denen jeweils zwei mit einem Sense-Kanal verbunden sind, ohne dass zwei Sense-Kanäle mit denselben beiden Elektroden verbunden sind. Zwei Sense-Kanäle teilen sich somit immer nur eine der beiden Elektroden, mit denen sie verbunden sind.

Die Erfindung beruht auf der Erkenntnis, dass sich mit jedem der drei Sense-Kanäle jeweils eine Art Elektrokardiogramm aufnehmen lässt, wobei sich die auf diese Weise gewonnenen drei Elektrokardiogramme, insbesondere hinsichtlich des auf einen gemeinsamen Bezugszeitpunkt bezogenen Auftretens voneinander entsprechenden Signalmerkmalen, unterscheiden. Durch Auswerten derartiger zeitlicher Verschiebungen lassen sich wertvolle zusätzliche Informationen über einen jeweiligen Herzzustand gewinnen, die einem einzelnen Elektrokardiogramm nicht zu entnehmen sind. Insbesondere erlaubt das Auswerten der zeitlichen Beziehung zwischen einander entsprechenden Signalmerkmalen in den verschiedenen Eingangskanälen eine verbesserte Tachykardieerkennung, eine verbesserte Einstellung des Stimulationsschwellwertes eines biventrikulären Herzschrittmachers, eine Effizienzkontrolle eines biventrikulären Herzschrittmachers und vor allem die Erkennung ventrikulärer Fibrillationen. Wenn die drei Sense-Kanäle jeweils ein Fernfeld der Myokardpotentiale erfasst und ein vierter Sense-Kanal zum Erfassen eines Nahfeldes der Myokardpotentiale vorgesehen ist, kann eine ventrikuläre Fibrillation daran erkannt werden, dass die Auslenkungen der drei Fernfeld-Signale sehr gering sind, während im Nahfeld-Kanal eine Erregung mit einer hohen Rate zu erkennen ist.

Aus dem Vorstehenden ergibt sich, dass die Signalverarbeitungseinheit vorzugsweise ausgebildet ist, ein erfasstes aktuelles Signalmerkmal eines Eingangssignals hinsichtlich des Zeitpunktes des Auftretens eines Formmerkmals in dem Zeitfenster mit dem Zeitpunkt des Auftretens eines entsprechenden Formmerkmals in einem anderen Eingangssignal oder in einem vorangegangenen Zeitfenster zu vergleichen. Im Bezug auf diesen Erfindungsaspekt sowie überhaupt in Bezug auf die gesamte Erfindung ist mit Formmerkmal ein spezielles Signalmerkmal gemeint, welches sich auf die Signalform bezieht. Im einfachsten Falle ist ein Formmerkmal eine Signalspitze. Komplexere Formmerkmale können mit Hilfe eines Schablonen-Ansatzes erfasst werden. Ein Signalmerkmal im Allgemeinen kann ein Formmerkmal sein, es könnte beispielsweise aber auch grundsätzlich ein Zeitmerkmal sein.

Besonders bevorzugt ist eine Ausführungsvariante, bei der die Signalverarbeitungseinheit ausgebildet ist, das Auftreten eines Amplitudenmaximums als Signalmerkmal sowie den Zeitpunkt von dessen Auftreten in dem Zeitfenster zu erfassen.

Das Triggersignal zum Auslösen des Zeitfensters kann ein beliebiges, wiederkehrendes Triggersignal sein. Es kann beispielsweise von einem Markerkanal stammen. Vorteilhafterweise ist die Signalverarbeitungseinheit jedoch ausgebildet, das Triggersignal aus einem der Eingangssignale derart abzuleiten, dass das Triggersignal durch ein Signalmerkmal dieses Eingangssignals, beispielsweise das Auftreten eines Amplitudenmaximums, ausgelöst wird. Das Auftreten eines Amplitudenmaximums ist ein Form- und damit auch ein Signalmerkmal eines Eingangssignals.

Mit Hilfe eines Loop-Recorders für die Eingangssignale ist es im Übrigen möglich, auch Zeitfenster zu verarbeiten, deren Beginn zeitlich vor dem Auftreten des jeweiligen Triggersignals liegt. Im Übrigen kann das Zeitfenster in Bezug auf das Triggersignal durch Angabe absoluter Zeitwerte definiert sein, oder durch Angabe relativer Zeitwerte, die auf die Dauer eines jeweiligen Herzzyklusses bezogen sind.

Ebenso kann der Zeitpunkt des Auftretens eines Signalmerkmals innerhalb des Zeitfensters in absoluten Zeitwerten in Bezug auf das Triggersignal erfasst werden, oder indem die relative zeitliche Lage des Zeitpunkts des Auftretens des Signalmerkmals in dem jeweiligen Zeitfenster erfasst wird. Der letzte Fall läuft darauf hinaus, dass der Zeitpunkt des Auftretens des Signalmerkmals nicht in absoluten Zeitwerten, sondern in Bezug auf die Dauer des Zeitfensters erfasst wird.

In Bezug auf das Triggersignal ist es vorteilhaft, wenn das Triggersignal aus einem Eingangssignal abgeleitet wird, welches ein Nahfeldpotiential repräsentiert.

Zu letzterem Zweck ist vorzugsweise ein vierter Eingangskanal als vierter Sense-Kanal vorgesehen, der mit mindestens einer Elektrode oder einem Anschluss für eine Elektrode verbunden ist, mit der sich das Nahfeld eines vierten, mit der Erregung des Herzgewebes einhergehenden elektrischen Potentials erfassen lässt. Die Signalverarbeitungseinheit ist in diesem Falle dazu ausgebildet, das Triggersignal aus dem Eingangssignal des vierten Sense-Kanal abzuleiten.

Die ersten drei Sense-Kanäle sind vorzugsweise Fernfeldkanäle und jeweils mit einer solchen Elektrode oder einem Anschluss für eine solche Elektrode verbunden, mit der sich im Betriebszustand des implantierbaren Elektrostimulationsgerätes jeweils ein Fernfeld eines mit einer Erregung des Herzgewebes einhergehenden elektrischen Potentials erfassen lässt.

In einer bevorzugten Ausführungsvariante sind die drei Eingangskanäle mit drei Elektrodenleitungsanschlüssen verbunden, nämlich einem Anschluss für eine proximale Defibrillationselektrode, beispielsweise eine atriale Schockelektrode, einem Anschluss für eine distale Defibrillationselektrode, beispielsweise eine ventrikuläre Schockelektrode, und dem Gehäuse des implantierbaren Kardioverters/Defibrillators, das somit ein aktives Gehäuse ist.

Zum Erfassen des ein Nahfeld repräsentierenden Eingangssignals über einen Nahfeld-Kanal ist vorzugsweise wenigstens eine bipolare, rechtsventrikuläre Elektrodenleitung vorgesehen, die eine ventrikuläre Ringelektrode und eine ventrikuläre Tip-Elektrode aufweist, über die das Nahfeld zu erfassen ist.

Vorzugsweise sind wenigstens drei Eingangskanäle Fernfeld-Kanäle, die mit je einer solchen Elektrode oder mit einem Anschluss für eine solche Elektrode verbunden sind, mit der im Betriebszustand des implantierbaren Elektrostimulationsgerätes jeweils ein Fernfeld eines mit einer Erregung des Herzgewebes (Myokard) einhergehenden elektrischen Potentials zu erfassen ist, so dass an den Eingangskanälen ein ein jeweiliges Fernfeld repräsentierendes Eingangssignal anliegt. Das implantierbare Elektrostimulationsgerät weist eine Signalverarbeitungseinheit auf, die mit den Eingangskanälen verbunden und ausgebildet ist, drei voneinander verschiedene Differenzsignale zu bilden, die sich aus der Differenz zweier an je einem Eingangskanal anliegenden Signalen ergeben, und für einen jeweiligen Herzzyklus für jedes der Differenzsignale einen jeweiligen Maximumamplitudenwert zu bilden, der die maximale Auslenkung des jeweiligen Differenzsignals innerhalb des jeweiligen Herzzyklusses repräsentiert.

Statt den Maximumamplitudenwert zu bilden, kann die Signalverarbeitungseinheit ausgebildet sein, die Amplitude (Auslenkung) jedes der Differenzsignale zu einem festen Bezugszeitpunkt innerhalb eines Herzzyklusses zu bestimmen und auszuwerten. Der Maximumamplitudenwert ist jedoch am aussagekräftigsten und am einfachsten zu bestimmen. Gegebenenfalls kann der Bezugszeitpunkt durch einen an sich bekannten Markerkanal vorgegeben werden, der Markersignale z.B. beim Erfassen einer P-Welle, einer T-Welle oder eine R-Zacke generiert.

Das Differenzsignal ist im speziellen Fall ein Signal, welches als Elektrokardiogramm darzustellen ist. In einer speziellen Ausführungsvariante ist die Signalverarbeitungseinheit daher ausgebildet, aus den Eingangssignalen von je zwei Eingangskanälen ein Elektrogramm abzuleiten - insgesamt also vorzugsweise drei Elektrokardiogramme. Mit Elektrogramm ist hier ein elektrisches Signal gemeint, welches als Elektrokardiogramm darstellbar ist. In diesem speziellen Fall ist die Signalverarbeitungseinheit ausgebildet, pro Herzzyklus je eine Amplitude für jedes der Elektrokardiogramme zu bestimmen. Diese Amplitude ist vorzugsweise die maximale Auslenkung des Elektrokardiogramms, also die Maximumamplitude, welche zum Zeitpunkt einer jeweiligen R-Zacke auftritt. Die R-Zacke ist eine Signalzacke, die in Folge der Depolarisierung des Myokards auftritt, welche zur Kontraktion des jeweiligen Herzmuskels führt.

Die Erfindung beruht auf der Erkenntnis, dass drei derart gewonnene Maximumamplituden einen Informationsgehalt besitzen, der sowohl beispielsweise eine Differenzierung ventrikulärer und supraventrikulärer Tachykardien als auch eine zuverlässige Erkennung von jeweiligen Kammerkontraktionen erlaubt.

In einer wegen ihrer Einfachheit besonders bevorzugten Ausführungsvariante ist die Signalverarbeitungseinheit ausgebildet, Betrag und Vorzeichen der Maximumamplitudenwerte im Verhältnis zueinander auszuwerten. Die drei Maximumamplitudenwerte ergeben einen 3-Tupel, der für den Ausgangsort einer Kammererregung charakteristisch ist und es daher erlaubt, eine rechtsseitige wie auch eine linksseitige Kammerkontraktion voneinander zu unterscheiden und außerdem eine stimulierte Kammerkontraktion von einer natürlichen und schließlich eine Kammerkontraktion ventrikulären Ursprungs von einer Kammerkontraktion supraventrikulären Ursprungs. Dies wird im Zusammenhang mit den Ausführungsbeispielen näher erläutert.

Neben einer Auswertung der 3-Tupel kann auch zum Vergleich der Maximalamplitudenwerte ein Schablonenansatz herangezogen werden. Dazu wird jeder erfasste Maximalamplitudenwert beispielsweise einer von drei Kategorien zugeordnet, nämlich kleine Amplitude, mittlere Amplitude oder große Amplitude. Auf diese Weise lassen sich sehr kompakte Beschreibungen der miteinander zu vergleichenden Maximalamplitudenwerte erzeugen, die auch einfach auszuwerten sind.

Die Erfindung beruht auf der Erkenntnis, dass die jeweilige Amplitude eines jeweiligen Differenzsignals als Betrag eines Vektors aufgefasst werden kann, der jeweils zusammen mit den beiden anderen Vektoren zu einem Summenvektor addiert werden kann, der insbesondere die Richtung der Erregungsausbreitung im Myokard zum jeweiligen Zeitpunkt beschreibt. Hierzu sind auch die Winkel zwischen den drei die Komponenten des Summenvektors bildenden Vektoren zu berücksichtigen. Diese Winkel sind durch die räumliche Anordnung der drei mit den drei Eingangskanälen verbundenen Elektroden in deren implantierten Zustand vorgegeben. Da diese räumliche Anordnung der Elektroden im implantierten Zustand weitestgehend gleichbleibend ist, sind die Winkel Konstanten, deren genauer Wert von untergeordneter Bedeutung ist, wenn die interessierende Information bereits durch Auswerten von Richtungsänderungen des Summenvektors gewonnen werden kann. Die Erfindung macht sich diese Erkenntnis, dass allein durch das Auswerten von Richtungsänderungen wesentliche Informationen gewonnen werden können, zunutze und ergänzt sie um die weitere Erkenntnis, dass im einfachsten Fall nur noch die Beträge der Amplituden und ihre Vorzeichen ausgewertet zu werden brauchen, um eine Richtungsänderung des Summenvektors zu detektieren. Dafür reicht es, die jeweiligen Amplituden zu einem bestimmten Zeitpunkt innerhalb des Herzzyklusses zu ermitteln, so dass pro Herzzyklus nur drei Amplitudenwerte zu bestimmen sind, nämlich für jedes der Differenzsignale einer. Eine weitere Erkenntnis besteht darin, dass der geeignetste Bezugszeitpunkt der Zeitpunkt des Auftretens der R-Zacke ist. Dieser ist aus doppeltem Grunde geeignet: Zum einen ist er leicht zu identifizieren, da er jedem Differenzsignal als Zeitpunkt des Auftretens der Maximumamplitude inhärent ist. Zum anderen kennzeichnet dieser Zeitpunkt eine jeweilige Kammerkontraktion. Daher kann von der Richtung des Summenvektors zu diesem Zeitpunkt in idealer Weise auf den Ursprungsort der Erregung zurückgeschlossen werden. Dies wiederum erlaubt es - wie schon gesagt - stimulierte von natürlichen Kammererregungen zu unterscheiden, linksventrikuläre Erregungen von rechtsventrikulären und supraventrikuläre Erregungen von ventrikulären Erregungen.

Ein weiterer, besonderer Vorteil der Erfindung besteht darin, dass sie sich mit relativ geringem Aufwand als Ergänzung eines an sich bekannten implantierbaren Kardioverters/Defibrillators realisieren lässt, indem zur Aufnahme der Eingangssignale Eingangskanäle vorgesehen werden, die mit ohnehin benötigten Elektroden verbunden sind, nämlich in einer bevorzugten Ausführungsvariante mit einer atriumnahen Schockelektrode, mit einer ventrikulären Schockelektrode und mit einer vom Gehäuse des implantierbaren Kardioverters/Defibrillators gebildeten Elektrode. In einer bevorzugten Ausführungsvariante ist die atriale Schockelektrode eine relativ großflächige, längsgestreckte proximale Defibrillationselektrode auf einer Elektrodenleitung und die ventrikuläre Schockelektrode ist eine ebenfalls längsgestreckte distale Defibrillationselektrode auf derselben Elektrodenleitung. Diese Ausführungsvariante ist je ein Eingangskanal mit einem Anschluss für die atriale Schockelektrode und die ventrikuläre Schockelektrode verbunden, während der dritte Eingangskanal direkt mit der vom aktiven Gehäuse des implantierbaren Kardioverters/Defibrillators gebildeten Elektrode verbunden ist.

Wie später noch ausführlich erläutert werden wird, ist ein solcher implantierbarer Kardioverter/Defibrillator (ICD) leicht so zu gestalten, dass er mit größerer Sicherheit als bekannte Geräte, beispielsweise ventrikuläre Tachykardien von supraventrikulären Tachykardien unterscheiden kann.

Ist das implantierbare Stimulationsgerät darüber hinaus auch noch als Drei- oder Mehrkammschrittmacher ausgebildet, der als biventrikulärer Schrittmacher in der Lage ist, sowohl den rechten als auch den linken Ventrikel zu stimulieren, ergibt sich der weitere Vorteil, dass dadurch ein Mehrkammerschrittmacher im Sinne der Erfindung leicht so ausgebildet werden kann, dass er Kammerkontraktionen aufgrund rechtsventrikulärer oder linksventrikulärer Stimulation voneinander und von natürlichen Kammerkontraktionen unterscheiden kann. Dies ist insbesondere im Zusammenhang mit der eingangs erläuterten Stimulationserfolgskontrolle (Capture Control) von großem Vorteil.

Wie zuvor bereits erläutert, reicht zur Umsetzung der Erfindung das implantierbare Elektrostimulationsgerät - sei es nun ein implantierbarer Kardioverter/Defibrillator oder ein Mehrkammerherzschrittmacher oder eine Kombination aus beidem - mit einer Signalverarbeitungseinheit zu versehen, die in zuvor genannter Weise mit Elektroden zu verbinden ist und die ausgebildet ist, Betrag und Vorzeichen der Maximumamplitudenwerte im Verhältnis zueinander auszuwerten.

In einer bevorzugten Ausführungsvariante weist das Elektrostimulationsgerät einen Speicher auf, in dem für jedes der Differenzsignale beziehungsweise jedes der Elektrokardiogramme ein Winkelwert vorzugsweise dauerhaft gespeichert ist, der es erlaubt, zusammen mit einer jeweiligen Amplitude eines jeweiligen Elektrokardiogramms beziehungsweise dem Maximumamplitudenwert des Differenzsignals einen Vektor zu bestimmen, der bei Vektoraddition mit entsprechenden Vektoren, die sich aus Winkeln und Amplituden der beiden jeweils anderen Elektrokardiogramme beziehungsweise Differenzsignale per Vektoraddition zu einem Summenvektor addieren lässt, dessen Richtung dann von der Signalverarbeitungseinheit auszuwerten ist. Mit Richtung des Summenvektors ist im vorliegenden Fall die vorzeichenbehaftete Ausrichtung des Vektors gemeint, das heißt Richtung und einschließlich Orientierung des Summenvektors.

Das implantierbare Elektrostimulationsgerät ist vorzugsweise dazu ausgebildet, eine mögliche Richtungsänderung des Summenvektors von Herzzyklus zu Herzzyklus (Beat to Beat) zu ermitteln, das heißt für jedes der Elektrokardiogramme beziehungsweise Differenzsignale pro Herzzyklus wenigsten einen Amplitudenwert zum gegebenen Bezugszeitpunkt zu bestimmen. Wie bereits ausgeführt, reicht es dabei, für jedes Elektrokardiogramm beziehungsweise Differenzsignal pro Herzzyklus genau einen Amplitudenwert zu bestimmen, und zwar vorzugsweise zum Zeitpunkt einer durch eine R-Zacke gekennzeichneten Kammerkontraktion, um die Erfindung in besonders einfacher und effizienter Weise umzusetzen. Um eine Änderung der Richtung des Summenvektors von Zyklus zu Zyklus zu ermöglichen, ist die Signalverarbeitungseinrichtung vorzugsweise mit einem Speicher verbunden, in dem die Richtung des Summenvektors für den jeweils vorangegangenen Herzzyklus zu speichern ist. Die Signalverarbeitungseinheit ist dabei vorzugsweise so ausgebildet, dass sie eine Richtungsänderung des Summenvektors kennzeichnendes Signal nur dann erzeugt, wenn der Summenvektor seine Richtungsänderung um mehr als einen vorgegebenen Toleranzwinkel ändert. In diesem Zusammenhang kann es vorteilhaft sein, wenn das implantierbare Stimulationsgerät so ausgebildet ist, dass die Richtungen der Summenvektoren mehrerer vorangegangener Herzzyklen zu speichern sind.

In einer besonders bevorzugten Ausführungsvariante ist die Signalverarbeitungseinheit so ausgebildet, dass sie auf vorgegebene Winkelbereiche zurückgreifen kann, die für bestimmte Erregungsorte charakteristisch sind. Anders als im vorangegangenen Fall prüft die Signalverarbeitungseinheit somit nicht nur, ob sich die Richtung des Summenvektors gegenüber dem der Richtung des Summenvektors aus dem vorangegangenen Herzzyklus der den vorangegangenen Herzzyklen geändert hat. Vielmehr prüft die Signalverarbeitungseinheit, ob die jeweils ermittelte Richtung des Summenvektors in einen von mehreren Winkelbereichen fällt, wobei einer dieser Winkelbereiche beispielsweise einer stimulierten rechtsventrikulären Kammerkontraktion zugeordnet ist, während ein anderer Winkelbereich einer stimulierten linksventrikulären Kammerkontraktion zugeordnet ist und ein dritter Winkelbereich einer Kammerkontraktion aufgrund natürlicher Reizleitung vom Atrium zum Ventrikel zugeordnet ist. Weitere Winkelbereiche können zum Beispiel typischen ektopischen Erregungsherden für die ventrikuläre Stimulation zugeordnet sein.

Um eine noch genauere Klassifikation, insbesondere von Tachykardien zu ermöglichen, oder um einen jeweiligen Bezugszeitpunkt innerhalb eines Herzzyklusses möglichst genau zu bestimmen, weist das Elektrostimulationsgerät vorzugsweise einen weiteren Eingangskanal auf, der mit einer Elektrode oder einem Anschluss für eine Elektrode verbunden ist, mit der im Betriebszustand des Elektrostimulationsgerätes ein Nahfeld eines mit einer Erregung des Herzgewebes einhergehenden elektrischen Potentials zu erfassen ist, so dass an dem weiteren Eingangskanal ein das Nahfeld repräsentierende Signal anliegt. Die Signalverarbeitungseinheit ist in diesem bevorzugten Fall auch mit dem weiteren Eingangskanal verbunden und ausgebildet, dass das Nahfeld repräsentierende Eingangssignal jeweils hinsichtlich einer Kammerkontraktion kennzeichnender Signalspitzen auszuwerten.

Um in einer bevorzugten Variante die jeweilige Herzrate zur Klassifikation von Tachykardien heranziehen zu können, ist die Signalverarbeitungseinheit in jedem Fall ausgebildet, den zeitlichen Abstand der ermittelten Signalspitzen auszuwerten und hieraus die Dauer eines jeweiligen RR-Intervalls oder eine Herzrate abzuleiten.

Zusätzlich kann die Signalverarbeitungseinheit ausgebildet sein, aus einer jeweiligen Signalspitze ein Markersignal abzuleiten.

Dieses Markersignal kann bei entsprechender Ausbildung der Signalverarbeitungseinheit dazu genutzt werden, die Amplitude (beziehungsweise Auslenkung) des Differenzsignals oder des Elektrokardiogramms für jeden Herzzyklus zu dem durch das jeweilige Markersignal vorgegebene Zeitpunkt zu bestimmen und die so gewonnenen Amplituden als Beträge der Komponenten eines Summenvektors zur Bestimmung der Richtung des Summenvektors auszuwerten.

Die Erfindung soll nun anhand von Ausführungsbeispielen mit Bezug auf die Figuren näher erläutert werden. Von den Figuren zeigt:
- Fig. 1:: die Skizze eines erfindungsgemäßen Einkammer-ICD mit angeschlossenen Elektrodenleitungen und Position der Elektroden im menschlichen Herzen,
- Fig. 2:: Elektrokardiogramme, wie sie mit der in Figur 1 abgebildeten Konstellation zu erfassen sind,
- Fig. 3:: eine Skizze, ähnlich Figur 1, jedoch in diesem Falle mit einem Zweikammer-ICD
- Fig. 4:: Elektrokardiogrammsignale, wie sie mit dem Zweikammer-ICD gemäß Figur 3 gewonnen werden können;
- Fig. 5:: eine Skizze eines Dreikammer-ICDs mit angeschlossenen, implantierten Elektroden;
- Fig. 6:: eine Ausführungsvariante, bei der die Eingangssignale der drei Fernfeldkanäle durch Auswerten der Differenzamplituden gebildet werden, die paarweise zwischen einem aktiven Gehäuse des Dreikammer-ICDs und einer ventrikulären Schockelektrode, der ventrikulären Schockelektrode und einer atrialen Schockelektrode und schließlich dem aktiven Gehäuse des ICDs und der atrialen Schockelektrode erfasst werden;
- Fig. 7:: eine Ausführungsvariante, bei der die Eingangssignale der drei Fernfeldkanäle durch Auswerten der Differenzamplituden gebildet werden, die paarweise zwischen einem aktiven Gehäuse des Dreikammer-ICDs und einer ventrikulären Schockelektrode, der ventrikulären Schockelektrode und einer atrialen Ring- oder Tipelektrode und schließlich dem aktiven Gehäuse des ICDs und der atrialen Ring- oder Tipelektrode erfasst werden;
- Fig. 8:: eine Ausführungsvariante, bei der die Eingangssignale der drei Fernfeldkanäle durch Auswerten der Differenzamplituden gebildet werden, die paarweise zwischen einer linksventrikulären Ring- oder Tipelektrode und einer ventrikulären Schockelektrode, der ventrikulären Schockelektrode und einer atrialen Ring- oder Tipelektrode und schließlich der linksventrikulären Ring- oder Tipelektrode und der atrialen Ring- oder Tipelektrode erfasst werden;
- Fig. 9:: eine Skizze zur Erläuterung der Bestimmung der Maximumamplitude im jeweiligen Elektrokardiogramm;
- Fig. 10:: eine Skizze zur Erläuterung, wie aus den gemäß Figur 5 ermittelten Amplitudenwerten eine Richtung der Erregungsausbreitung ermittelt werden kann;
- Fig. 11:: eine kompakte Darstellung der gemäß Figur 5 ermittelten Amplitudenwerte;
- Fig. 12:: eine Skizze, ähnlich Figur 6, jedoch für einen für eine ventrikuläre Tachykardie typischen Fall;
- Fig. 13:: eine graphische Darstellung eines Klassifikationsalgorithmus, wie er durch die Signalverarbeitungseinheit eines erfindungsgemäßen implantierbaren Stimulationsgerätes verwirklicht ist;
- Fig. 14:: eine Skizze, ähnlich Figur 1, jedoch in diesem Falle mit einem Dreikammer-ICD;
- Fig. 15:: Elektrokardiogrammsignale, wie sie mit dem Dreikammer-ICD gemäß Figur 10 gewonnen werden können;
- Fig. 16:: eine Skizze, ähnlich Figur 6, jedoch hier für den Fall erfolgreicher biventrikulärer Stimulation;
- Fig. 17:: eine kompakte Darstellung der Amplituden, wie sie sich im Fall einer erfolgreichen biventrikulären Stimulation ergeben; ähnlich Figur 7;
- Fig. 18:: eine Skizze, ähnlich Figur 6, jedoch für den Fall erfolgreicher rechtsventrikulärer Stimulation aber erfolgloser linksventrikulärer Stimulation;
- Fig. 19:: eine kompakte Darstellung der Amplituden, ähnlich Figur 7, für den Fall eines rechtsventrikulären Stimulationserfolges und eines linksventrikulären Capture-Verlusts, wie er in Figur 12 dargestellt ist;
- Fig. 20:: eine Skizze, ähnlich Figur 6, jedoch hier für den Fall, dass die Stimulation nur im linken Ventrikel erfolgreich ist;
- Fig. 21:: die der Skizze in Figur 14 zugrunde liegenden Amplituden der Elektrokardiogramme in einer kompakten Darstellung, ähnlich Figur 7;
- Fig. 22:: ein Bericht über rechts- beziehungsweise linksseitige Capture Verluste, hier mit Hilfe eines erfindungsgemäßen Dreikammer-ICDs zu erstellen und telemetrisch an ein Homemonitoring Center zu übermitteln ist;

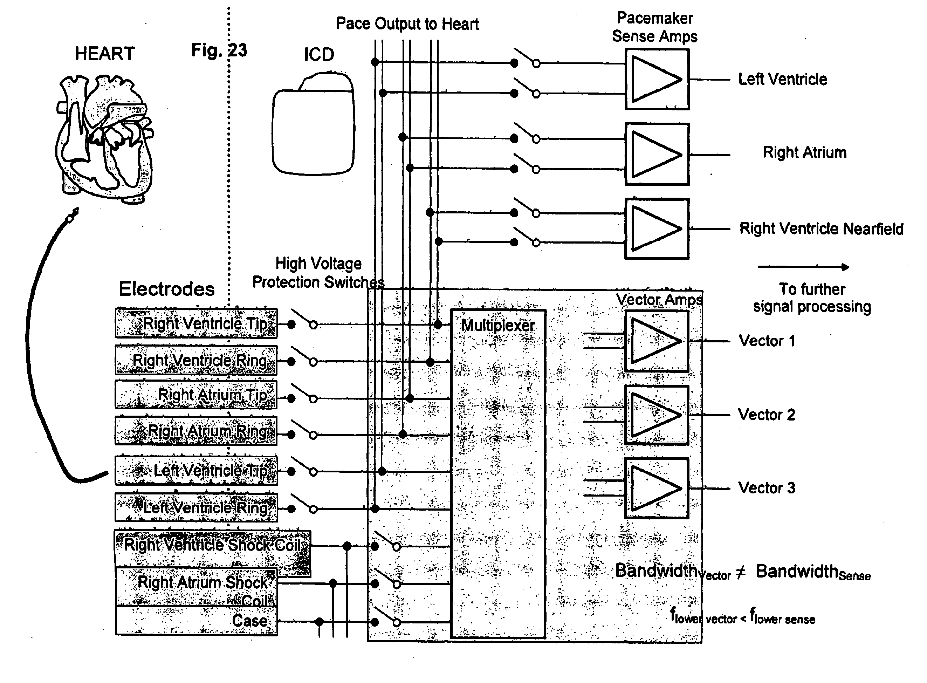

- Fig. 23:: eine schematische Darstellung der Eingangskanäle eines erfindungsgemäßen ICDs;
- Fig. 24:: die Eingangssignale eines Nahfeldkanals als Triggerkanal sowie von drei Fernfeldkanälen, wobei durch ein vom Nahfeldkanal abgeleitetes Triggersignal ein Zeitfenster bestimmt wird, welches in zeitlicher Hinsicht vor dem Triggersignal beginnt und nach dem Triggersignal endet; diese Ausführungsvariante erfordert einen Loop-Recorder;
- Fig. 25:: zum einen eine kompakte Darstellung der Maximalamplituden der Fernfeldsignale gemäß Figur 24 sowie zum anderen eine kompakte Darstellung des Zeitversatzes zwischen dem jeweiligen Zeitpunkt des Auftretens einer Maximalamplitude in Bezug auf den Zeitpunkt des Triggersignals;
- Fig. 26:: die Eingangssignale eines Nahfeldkanals als Triggerkanal sowie von drei Fernfeldkanälen, wobei durch ein vom Nahfeldkanal abgeleitetes Triggersignal ein Zeitfenster bestimmt wird, welches in zeitlicher Hinsicht nach dem Triggersignal beginnt und nach dem Triggersignal endet; und
- Fig. 27:: zum einen eine kompakte Darstellung der Maximalamplituden der Fernfeldsignale gemäß Figur 26 sowie zum anderen eine kompakte Darstellung des Zeitversatzes zwischen dem jeweiligen Zeitpunkt des Auftretens einer Maximalamplitude in Bezug zum Zeitpunkt des Triggersignals.

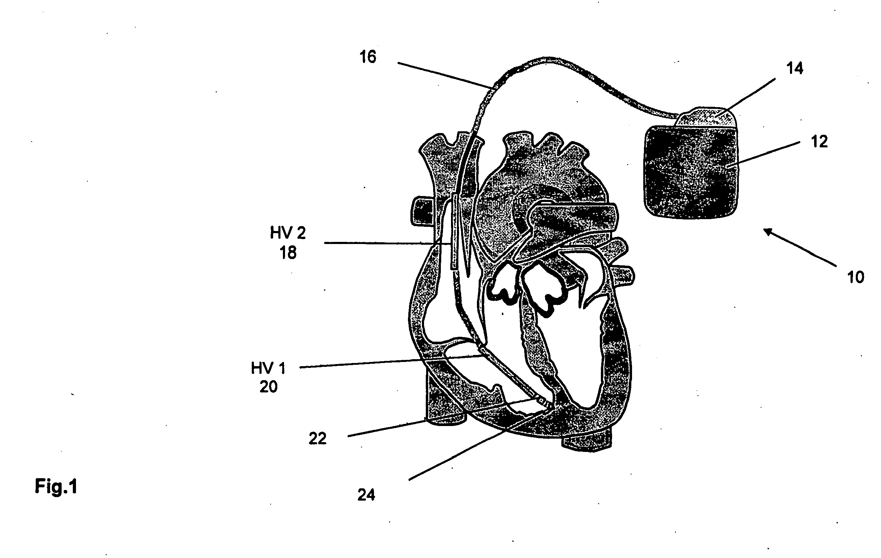

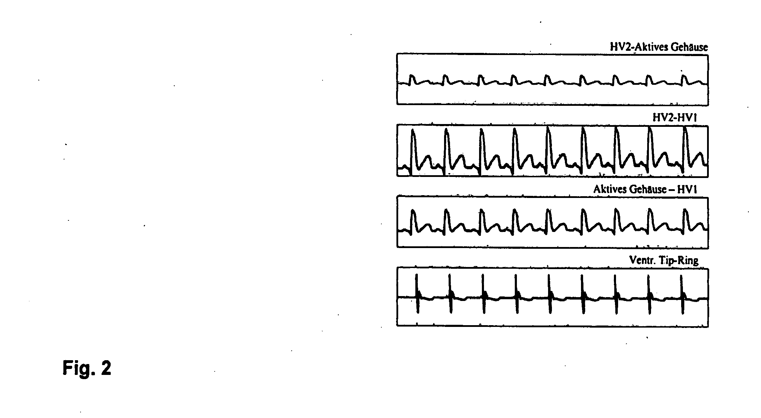

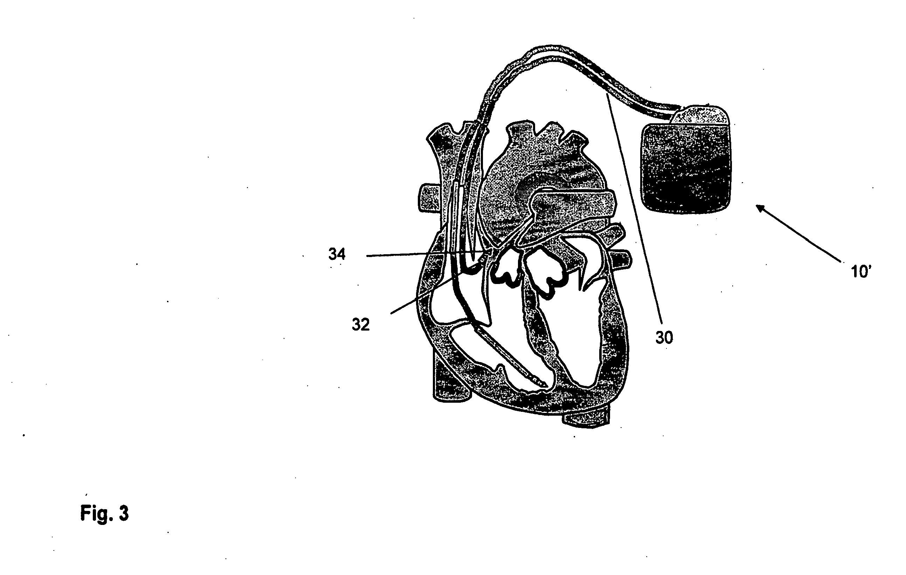

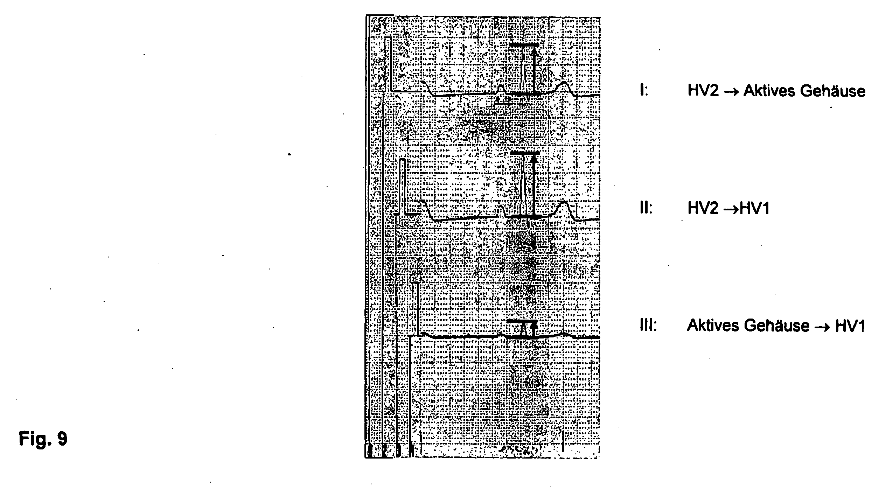

Die Figuren 1 bis 13 dienen der Erläuterung der Erfindung im Hinblick auf implantierbare Kardioverter/Defibrillatoren (ICDs) in Form von Einkammer-ICDs (Figuren 1 und 2) und Zweikammer-ICDs (Figuren 3 bis 8) und die Auswertung der mit diesen ICDs gewonnenen Signalamplituden im Hinblick auf die Klassifikation von ventrikulären Tachyarrhythmien (Figuren 9 bis 13).

Ziel dieser Auswertung ist es, eine bisher übliche, hohe Anzahl von ungewollten ICD-Interventionen aufgrund von supraventrikulären Tachykardien (SVT) zu reduzieren.

Bekannte Einkammer-ICDs analysieren ventrikuläre Intervalländerungen (RR-Intervalle) und verwenden bekannte Onset- und RR-Stabilitätskriterien zur Therapieinhibierung lediglich im Fall von Sinustachykardien und Vorhofflimmern. Andere bekannte Unterscheidungskriterien sind Morphologiekriterien zur Unterscheidung zwischen supraventrikulären und ventrikulären Tachykardien.

Zu bekannten Zweikammer-ICDs werden zusätzlich atriale Intervalle zur Verbesserung der Unterscheidung zwischen ventrikulären Tachykardien und supraventrikulären Tachykardien ausgewertet.

Wie die Unterscheidung verschiedener ventrikulärer Tachykardien mit einem erfindungsgemäßen, implantierbaren Elektrostimulationsgerät verbessert werden kann, soll nun im Folgenden näher erläutert werden.

Figur 1 zeigt ein geeignetes Elektrostimulationsgerät in Form eines Einkammer-ICDs 10. Der Einkammer-ICD 10 besitzt ein aktives Gehäuse 12, also ein Gehäuse mit einer elektrisch leitenden Oberfläche, die eine Elektrode bildet.

An einem Header 14 des ICDs 10 ist eine Elektrodenleitung 16 angeschlossen, die eine langgestreckte proximale Schockelektrode 18 und eine ebenfalls lang erstreckte distale Schockelektrode 20 sowie eine vergleichsweise kleinflächige Ringelektrode 22 und eine ebenfalls kleinflächige Tipp-Elektrode 24 trägt. Die Tipp-Elektrode 24 befindet sich dabei am distalen Ende der Elektrodenleitung 16.

In Figur 1 ist die Lage der Elektroden im implantierten Zustand dargestellt. Die proximale Schockelektrode 18 befindet sich im implantierten Zustand im unteren Ende der Vena Cava Superior im Bereich des Übergangs zum Atrium. Die distale Schockelektrode befindet sich im Ventrikel eines Herzens, die ventrikuläre Ringelektrode 22 und die ventrikuläre Tipp-Elektrode 24 sind im Bereich des Apex des Ventrikels des Herzens angeordnet.

Mit der in Figur 1 dargestellten Anordnung lassen sich die in Figur 2 dargestellten Elektrokardiogramme gewinnen, die jeweils als Differenzsignal den Verlauf der Potentialdifferenz zwischen zwei Elektroden darstellen, nämlich in Figur 2a das Differenzsignal, das sich als Differenz aus den an der proximalen Schockelektrode 18 und dem aktiven Gehäuse 12 anliegenden Potentialen ergibt. In Figur 2b ist das Differenzsignal dargestellt, welches sich aus den Verläufen der an den beiden Schockelektroden 18 und 20 anliegenden elektrischen Potentiale ergibt. Figur 2c zeigt das Differenzsignal, welches sich aus den am aktiven Gehäuse 12 und der distalen Schockelektrode 20 anliegenden Potentialen ergibt.

Die Differenzsignale in den Figuren 2a bis 2c stellen dabei jeweils Fernfeldsignale dar.

In Figur 2d ist schließlich der Verlauf der Potentialdifferenz zwischen der ventrikulären Ringelektrode 22 und der ventrikulären Tipp-Elektrode 24 dargestellt. Dieses Differenzsignal ist ein Nahfeldsignal.

Während die drei Schockelektroden, nämlich das aktive Gehäuse 12, die proximale Schockelektrode 18 und die distale Schockelektrode 22 Differenzsignale aufnehmen, die das Fernfeld der ventrikulären Erregung repräsentieren, wird über das von der ventrikulären Ringelektrode 22 der ventrikulären Tipp-Elektrode 24 gebildete Elektrodenpaar ein Differenzsignal aufgenommen, welches das Nahfeld der ventrikulären Erregung repräsentiert. Diese Differenzsignale sind daher Spike-förmige Signale, die für die Intervall-Klassifikation (also die Bestimmung des RR-Intervalls beziehungsweise der Herzrate) besonders geeignet sind und daher auch bei bekannten implantierbaren Stimulationsgeräten bereits ausgewertet werden.

Die mit Hilfe der drei Schockelektroden aufgenommenen drei Differenzsignale repräsentieren hingegen die Fernfelder der mit der ventrikulären Kontraktion einhergehenden elektrischen Potentiale und sind daher mit jenen Signalen vergleichbar, die für ein klassisches Oberflächenelektrokardiogramm mit Hilfe von auf den Körper aufgeklebten Elektroden gewonnen werden. Die drei Schockelektroden bilden wenigstens näherungsweise ein gleichseitiges Dreieck und entsprechen somit der Modellvorstellung nach Einthoven.

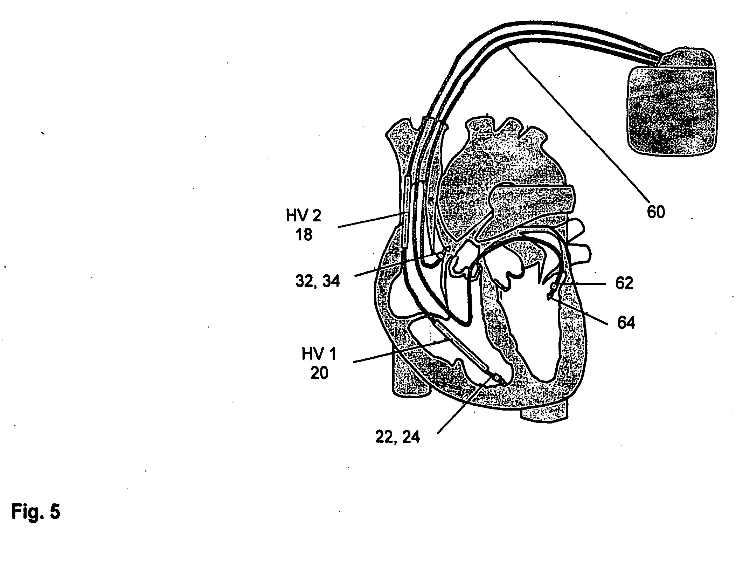

Figur 5 zeigt einen Dreikammer-ICD, der neben den bereits mit Bezug auf Figuren 1 und 3 beschriebenen Elektroden eine dritte Elektrodenleitung 60 aufweist, die als linksventrikuläre Elektrodenleitung über den Koronar Sinus und einer vom Koronar Sinus abzweigenden Lateralvene in die Peripherie des linken Ventrikels geführt ist und die an ihrem Ende eine linksventrikuläre Ringelektrode 62 und eine linksventrikuläre Spitzenelektrode 64 aufweist.

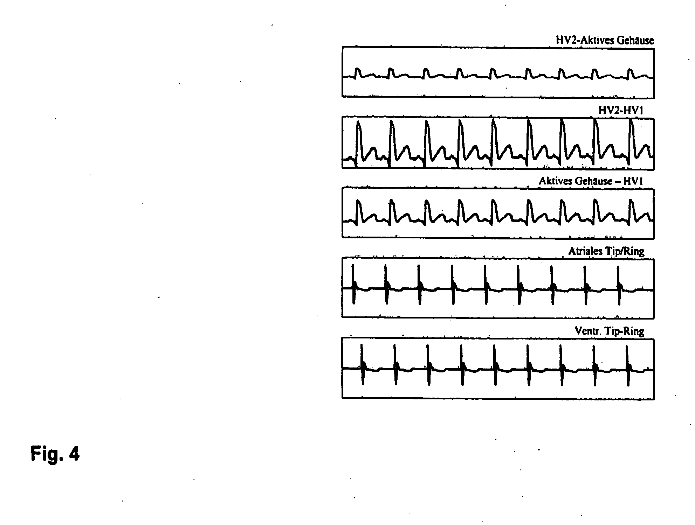

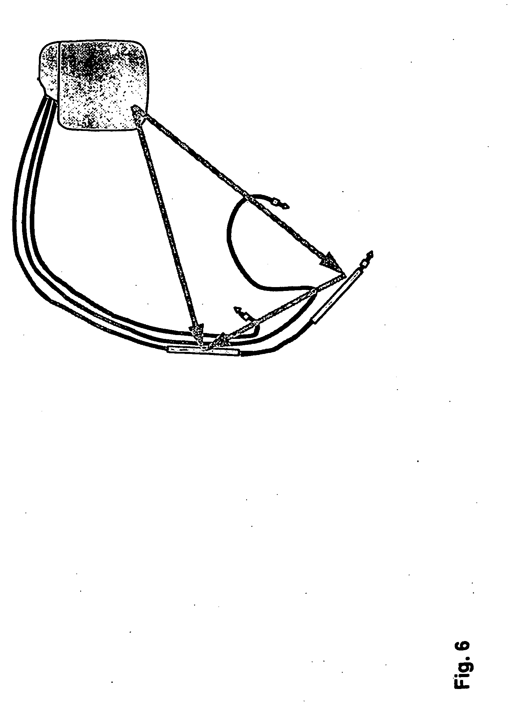

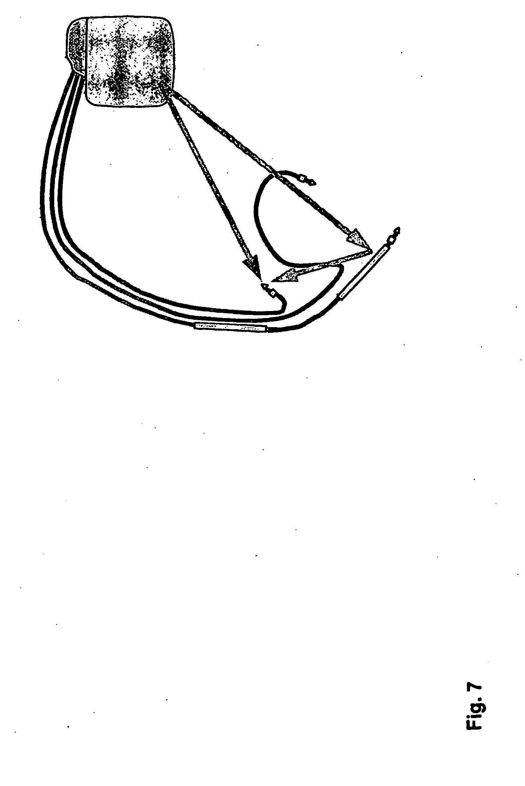

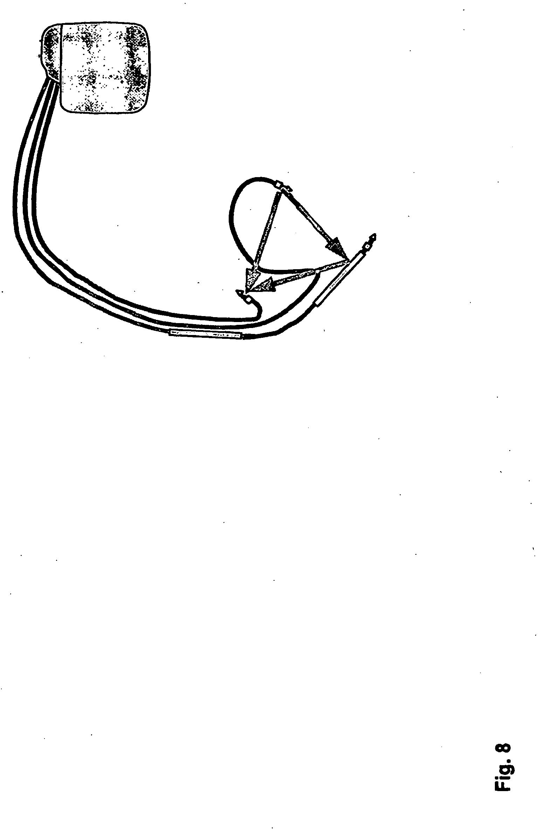

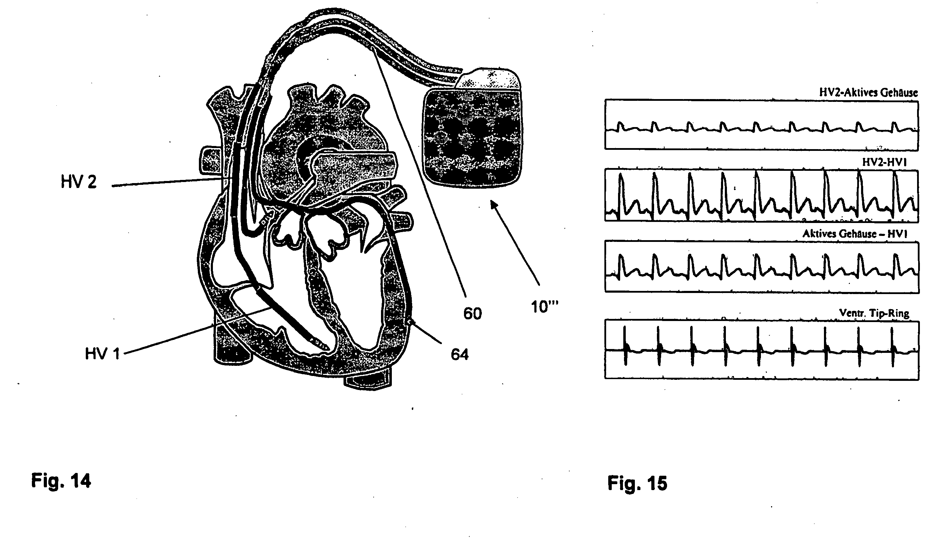

Ein Dreikammer-ICD der in Figur 5 dargestellten Art erlaubt es, die Differenzsignale für die drei Fernfeldkanäle auf verschiedene Art und Weise zu ermitteln, wie dies in den Figuren 6, 7 und 8 dargestellt ist. Dabei entspricht die Konfiguration in Figur 6 im Ergebnis derjenigen, die bereits mit Bezug auf die Figuren 1 und 2 erläutert wurde, während die Konfiguration in Figur 7 der Konfiguration entspricht, die bereits mit Bezug auf Figuren 3 und 4 erläutert wurde. Die Konfiguration in Figur 8 ähnelt der Konfiguration aus Figuren 3, 4 und 7, mit Ausnahme der Tatsache, dass als dritte Elektrode nicht das aktive Gehäuse des ICDs verwendet wird, sondern die linksventrikuläre Ring- oder Tipelektrode. Eine solche Konfiguration, wie sie in Figur 8 dargestellt ist, wird mit Bezug auf Figuren 14 und 15 weiter unten noch einmal näher erläutert.

Mit Bezug auf die folgenden Figuren 9 bis 13 soll nun erläutert werden, wie allein durch Auswerten der Maximumamplituden der drei Differenzsignale (siehe Figuren 2a bis 2c und 4a bis 4c) eine Information über den Ursprungsort beispielsweise einer Tachykardie gewonnen werden kann. Zur Erläuterung dessen wird auf die bekannte Theorie von Einthoven zurückgegriffen. Die Beträge der drei Maximumamplituden können nämlich als Beträge von Vektoren im an sich bekannten Einthovenschen Dreieck verstanden werden, welche vektoriell addiert und zur Berechnung eines Summenvektors herangezogen werden können, der die Richtung der Erregungsausbreitung im Myokard wiedergibt.

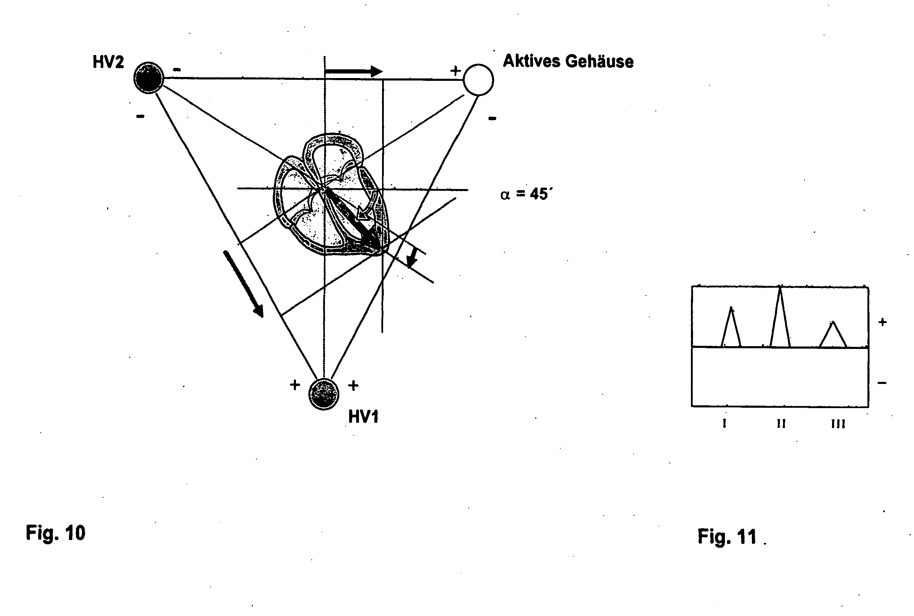

Figur 9 zeigt zunächst die Bestimmung der Maximumamplitude eines jeweiligen Differenzsignals. Die Maximumamplitude tritt genau einmal pro Herzzyklus auf und zwar zum Zeitpunkt der R-Zacke, das heißt also zum Zeitpunkt der Kontraktion des Herzgewebes infolge der Depolarisation der Herzmuskelzellen. Aus den drei Differenzsignalen werden somit pro Herzzyklus drei Amplitudenwerte gewonnen, die, wie in Figur 10 dargestellt, die Beträge dreier Vektoren 50, 52 und 54 darstellen, die sich per Vektoraddition zu einem Summenvektor 56 addieren lassen. Für die Vektoraddition ist grundsätzlich die Berücksichtigung der Winkel zwischen den Vektoren 50, 52 und 54 notwendig. Diese Winkel sind durch die ein Dreieck aufspannende Lage der drei Schockelektroden 12, 18 und 20 vorgegeben. In einem ideal gleichseitigen Dreieck beträgt jeder der Winkel 60°. Wie sich aus dem folgenden ergeben wird, ist die Erkenntnis der genauen Größe der Winkel zwischen den einzelnen Vektoren 50, 52 und 54 nicht unbedingt erforderlich, um dennoch eine hinreichend genaue, qualitative Aussage über die Richtung der Erregungsausbreitung im Myokard aus den Vorzeichen und Beträgen der drei Maximumamplituden abzuleiten, die aus den mit den drei Schockelektroden 12, 18 und 20 aufgenommenen Differenzsignalen abzuleiten sind.

Eine kompakte Darstellung der drei Maximumamplituden ist in Figur 11 wiedergegeben. In Zahlen ausgedrückt können die Beträge und Vorzeichen der drei Maximumamplituden auch zu einem 3-Tupel zusammengefasst werden, der jeweils die Richtung der Erregungsausbreitung hinreichend genau repräsentiert.

Die Figuren 9 bis 11 zeigen typische Amplituden bei einer natürlichen Erregungsausbreitung vom Atrium zum Ventrikel über den AV-Knoten eines Herzens.

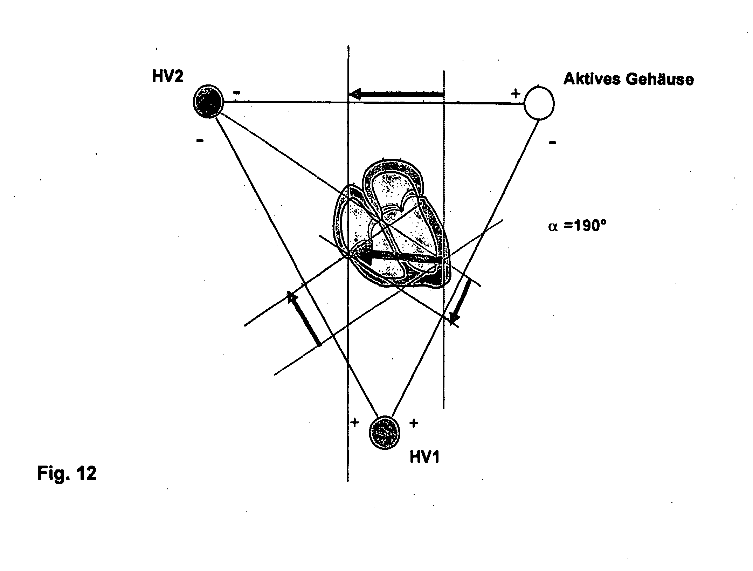

Figur 12 zeigt, wie stark sich die drei Maximumamplituden ändern, wenn die ventrikuläre Erregung ihren Ursprung in einem ektopischen Erregungsherd im rechten Ventrikel hat, wie dies beispielsweise bei einer ventrikulären Tachykardie der Fall ist. Es ist zu erkennen, wie sich die Richtung des Summenvektors (ausgedrückt durch einen Winkel α) um 145° geändert hat.

Im Sinne der mit den Figuren 10 und 12 eingeführten Winkeldefinition für den Winkel α erstreckt sich der Summenvektor bei normalem Sinusrhythmus in eine Richtung gekennzeichnet durch einen Winkel α zwischen 30° und 90°.

Da die Ausbreitung der ventrikulären Erregung im Falle supraventrikulärer Tachykardien (SVT) ebenfalls vom AV-Knoten ausgeht, beträgt α auch im Falle supraventrikulärer Tachykardien etwa 30° bis 90°. Dies gilt insbesondere für solche supraventrikulären Tachykardien wie Vorhofflattern, Vorhofflimmern, stabile, atriale Reentry-Tachykardie, AV-Knoten Reentry-Tachykardien oder das Wolf-Parkinson-White-Syndrom.

Im Falle ventrikulärer Tachykardien liegt der Ursprungsort der Tachykardie im Ventrikel. Ausgehend von einem arrhythmogenen Ursprungsort breitet sich die Erregungsfront im Myokard des Ventrikels in veränderter Richtung aus. Diese veränderte Erregungsausbreitungsrichtung spiegelt sich in einer Richtungsänderung des Summenvektors wieder. Diese Richtungsänderung spiegelt sich wiederum in einem veränderten Winkel α wieder (siehe Figur 12). Eine derartige Winkelveränderung tritt bei einer monomorphen ventrikulären Tachykardie oder bei einer polymorphen ventrikulären Tachykardie unabhängig von der Frequenz des Atriums auf.

Im Falle einer anfallsartig auftretenden ventrikulären Tachykardie ändert sich die Richtung des Summenvektors schlagartig mit Eintreten (onset) der Tachykardie. Eine ventrikuläre Extrasystole führt ebenfalls zu einer Richtungsänderung des Summenvektors, und zwar genau für einen Herzzyklus, in dem die ventrikuläre Extrasystole auftritt. Eine anhaltende ventrikuläre Tachykardie erzeugt hingegen eine dauerhafte Veränderung der Richtung des Summenvektors über viele Herzzyklen. Eine stabile ventrikuläre Reentry-Tachykardie mit einer 1:1 retrograden Leitung (vom Ventrikel zum Atrium) führt zu einer Änderung der Richtung des Summenvektors um näherungsweise 180°.

Allerdings führt auch eine stimulierte Kammerkontraktion (mit einer üblichen, im Apex des Ventrikels angeordneten Stimulationselektrode) zu einer ähnlichen Richtung des Summenvektors. Dies ist beim Bestimmen einer Tachykardie zu berücksichtigen und kann gleichzeitig zur Stimulationserfolgskontrolle (Capture Control) genutzt werden, wie nachfolgend noch beschrieben wird.

Um Tachykardien möglichst zuverlässig durch die Signalverarbeitungseinheit automatisch klassifizieren zu können, ist die Signalverarbeitungseinheit in einer bevorzugten Ausführungsvariante so ausgebildet, dass sie nicht nur eine Analyse der Richtung des Summenvektors durch Auswerten des beschriebenen 3-Tupels ausführen kann, sondern darüber hinaus auch bekannte Unterscheidungskriterien berücksichtigt.

Dazu kann es besonders von Vorteil sein, wenn der ICD ein Zweikammer-ICD ist, wie er in Figur 3 abgebildet ist. Dieser Zweikammer-ICD 10' ist mit einer zweiten Elektrodenleitung 30 verbunden, die der Stimulation des Atriums dient und die an ihrem distalen Ende eine atriale Ringelektrode 32 und eine atriale Tipp-Elektrode 34 besitzt. Über diese beiden atrialen Elektroden 32 und 34 ist neben den vier bereits in Figur 2 abgebildeten Differenzsignalen ein fünftes Differenzsignal zu gewinnen, welches sich aus den im Atrium zu erfassenden Potentialen ergibt und deren Nahfeld wiederspiegelt.

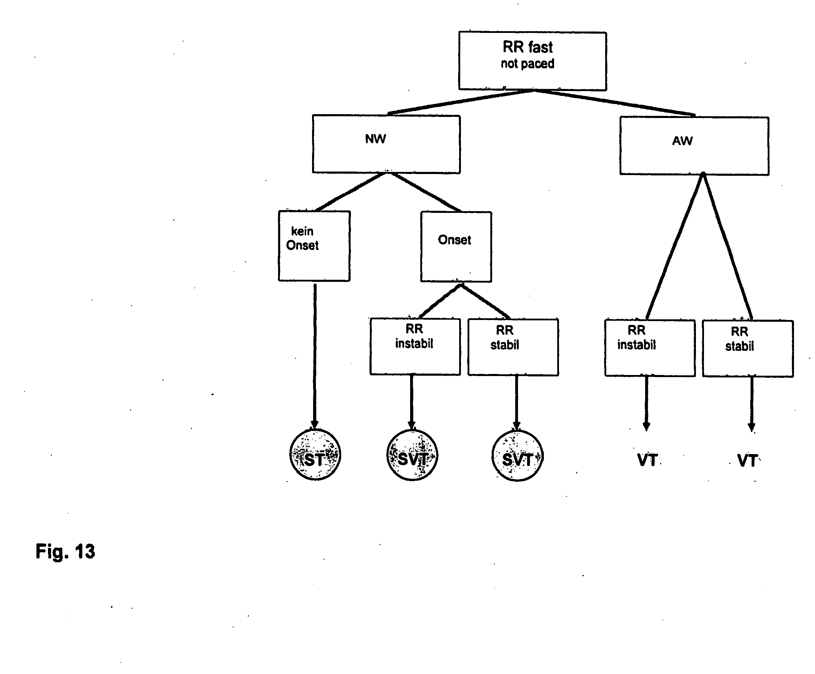

Figur 13 illustriert die Funktionsweise einer Signalverarbeitungseinheit, die zur Klassifikation von ventrikulären Tachykardien sowohl die Richtung des Summenvektors als auch Ratenkriterien berücksichtigt, wie sie mit Elektrodenpaaren wie der ventrikulären Ringelektrode 22 und der ventrikulären Tipp-Elektrode 24 beziehungsweise der atrialen Ringelektrode 32 und der atrialen Ringelektrode 34 zu gewinnen sind.

Wie Figur 13 zu entnehmen ist, ist der Ausgangpunkt für eine Klassifikation der ventrikulären Tachykardie eine hohe ventrikuläre Rate (RR-Rate). Die Signalverarbeitungseinheit ermittelt in diesem Fall zunächst durch Auswerten des oben beschriebenen 3-Tupels, ob sie die Richtung der Erregungsausbreitung (hierzu muss - wie zuvor ausgeführt - nicht erst der Summenvektor berechnet werden) in einem Normwinkelbereich (NW) oder in einem abnormen Winkelbereich (AW) befindet.

Falls die Signalverarbeitungseinheit ermittelt, dass sich die Richtung der Erregungsausbreitung im Ventrikel in einem abnormen Winkelbereich (AB) befindet, prüft die Signalverarbeitungseinheit weiterhin, ob die ventrikuläre Rate (RR-Rate) stabil ist oder instabil. In beiden Fällen wird eine ventrikuläre Tachykardie detektiert.

Ermittelt die Signalverarbeitungseinheit, dass sich die Richtung der Erregungsausbreitung im Normwinkelbereich (NW) befindet, prüft die Signalverarbeitungseinheit weiter, ob sich die ventrikuläre Rate (RR-Rate) allmählich verändert hat (kein onset) oder schlagartig verändert hat (onset). Im ersten Fall (kein onset) detektiert die Signalverarbeitungseinheit eine Sinustachykardie.

Im zweiten Fall (schlagartiges Eintreten der Tachykardie, onset) prüft die Signalverarbeitungseinheit, ob die ventrikuläre Rate instabil oder stabil ist. In beiden Fällen ermittelt die Signalverarbeitungseinheit eine supraventrikuläre Tachykardie (SVT).

Die in Abbildung 13 verwendeten Abkürzungen bedeuten im Einzelnen:
NW Normwinkelbereich
AW abnormer Winkelbereich
ST Sinus Tachykardie
SVT supraventrikulare Tachykardie
VT ventrikuläre Tachykardie.

Wie bereits angedeutet, kann eine Auswertung der Richtung der Erregungsausbreitung auch in sinnvoller Weise zur Stimulationserfolgskontrolle erfolgen. Dies ist insbesondere bei biventrikulären Stimulationsgeräten, also beispielsweise bei Dreikammer-ICDs von besonderem Vorteil.

Ein solcher Dreikammer-ICD ist in Figur 14 abgebildet. Im Unterschied zu den in Figuren 1 und 2 abgebildeten Ein- und Zweikammer-ICDs ist der Dreikammer-ICD 10''' aus Figur 14 mit einer dritten Elektrodenleitung 60 ausgestattet, die zur Stimulation des linken Ventrikels dient und üblicher Weise über den Koronar Sinus und eine vom Koronar Sinus abzweigende Lateralvene eingeführt ist. Die linksventrikuläre Elektrodenleitung 60 besitzt an ihrem distalen Ende eine linksventrikuläre Tipp-Elektrode 64.

Die in Figur 15 abgebildeten, mit dem Dreikammer-ICD gemäß Figur 14 aufzunehmenden Differenzsignale entsprechen denjenigen, die bereits im Zusammenhang mit Figur 2 erläutert wurden.

Wie bereits erwähnt, kann aus der Richtung der Erregungsausbreitung abgelesen werden, ob eine Kammerkontraktion auf eine erfolgreiche Stimulation zurückgeht.

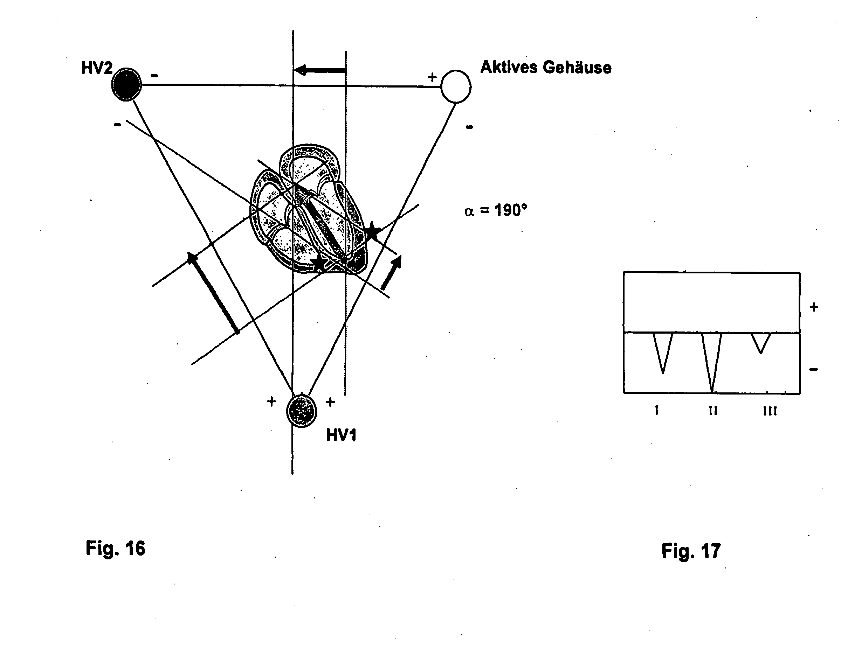

Dies sei mit Bezug auf Figur 16 näher erläutert. Bei einem stimulierten Herz beginnt die elektrische Erregung im Herzen an der Stelle der Stimulationselektrode. Von dort aus breitet sich die Erregungsfront über die Ventrikel aus. Das bedeutet, dass die Erregungsausbreitung und damit die Richtung des Summenvektors eine andere ist, als bei einer nicht stimulierten Herzaktion, bei der die Erregung vom AV-Knoten ausgeht.

Bei einer biventrikulären Stimulation des Herzens wird sich, ausgehend von den beiden stimulierten Ursprungsorten, die Erregungsfront in veränderter Richtung ausbreiten. Diese veränderte Erregungsausbreitungsrichtung spiegelt sich in einem anderen Winkel des Summenvektors wieder. Bei einer erfolgreichen biventrikulären Stimulation ergibt sich ein Winkel, wie er in Figur 16 dargestellt ist und der zu solchen Maximumamplituden der Differenzsignale führt, wie sie in Figur 17 in kompakter Form dargestellt sind.

Die Darstellung in Figur 17 zeigt die Polarität der einzelnen Maximumamplituden und deren Amplitudenverhältnis zueinander. Darin ist mit I die Maximumamplitude des Differenzsignals bezeichnet, welches zwischen dem aktiven Gehäuse 12 und der proximalen Schockelektrode 18 gewonnen wurde, mit II die Maximumamplitude des Differenzsignals, welches zwischen der proximalen Schockelektrode 18 und der distalen Schockelektrode 20 aufgezeichnet wurde und mit III die Maximumamplitude des Differenzsignals, welches zwischen der distalen Schockelektrode 22 und dem aktiven Gehäuse 12 erfasst wurde.

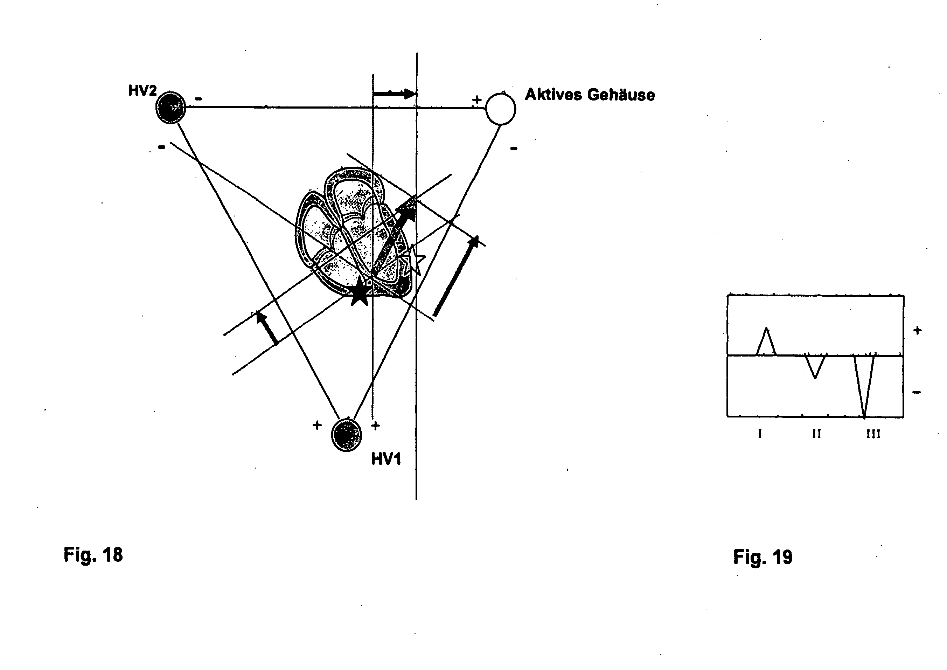

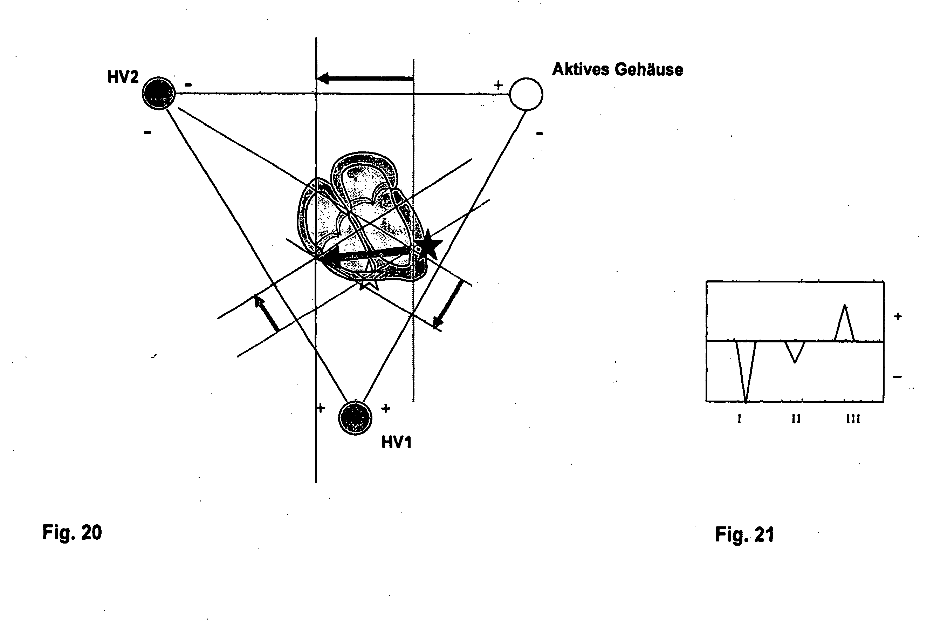

In Figur 16 und in den nachfolgenden Figuren 18 und 20 kennzeichnet ein ausgefüllter fünfzackiger Stern den Ort einer effektiven Stimulation, während ein nicht ausgefüllter (weißer) fünfzackiger Stern den Ort einer ineffektiven (also nicht wirksamen) Stimulation bezeichnet.

Figur 18 und die zugehörige Kompaktdarstellung der Maximumamplituden der Differenzsignalen zeigen, wie sich eine ineffektive Stimulation im linken Ventrikel auf die Richtung der Erregungsausbreitung (siehe Figur 18) und die Beträge sowie die Vorzeichen der Maximumamplituden (siehe Figur 19) auswirkt.

Entsprechendes stellen die Figuren 20 und 21 für den Fall der ineffektiven rechtsventrikulären Stimulation dar.

Bemerkenswert ist, dass die charakteristischen Winkeländerungen des Summenvektors infolge der veränderten Ausbreitungsrichtung bereits an den Vorzeichen der einzelnen Maximumamplituden abzulesen sind.

Ein besonderer Vorteil der hier vorgestellten Stimulationserfolgskontrolle auf Basis der Auswertung der Maximumamplituden der Differenzsignale besteht darin, dass diese Auswertung und damit das Feststellen eines Stimulationserfolges erfolgen kann, ohne dass dazu die Stärke der Stimulationsimpulse verändert werden muss.

Bei herkömmlichen Stimulationsgeräten mit automatischer Stimulationsstärkenanpassung wird die Stärke der Stimulationsimpulse schrittweise für so lange verringert, bis ein mangelnder Stimulationserfolg festzustellen ist. Bei der Stimulationserfolgskontrolle auf Basis der Auswertung der Maximumamplituden der Differenzsignale ergibt sich der weitere Vorteil, dass die Schockelektroden und nicht die Stimulationselektroden zur Stimulationserfolgskontrolle herangezogen werden. Außerdem ist die Erfassung des Stimulationserfolges auf Basis der Auswertung der Maximumamplituden unabhängig von eingestellten Schrittmacher-Parametern.

Wie sich aus den Figuren 18 und 20 ergibt, kann die Signalverarbeitungseinheit zur Stimulationserfolgskontrolle so ausgebildet sein, dass sie auf eine plötzliche Veränderung der Erregungsausbreitungsrichtung anspricht. Je nachdem ob diese schlagartige Richtungsänderung eine Winkeländerung nach links oder nach rechts ist, ergibt sich für die Signalverarbeitungseinheit, in welcher der Kammern der Capture-Verlust aufgetreten ist.

Sollte ein Capture-Verlust festgestellt werden, ist das Stimulationsgerät vorzugsweise dazu ausgebildet, die Stimulationsstärke für die jeweilige Kammer schrittweise (beispielsweise in Schritten von 0,1 Volt) zu erhöhen, bis sich wieder eine Erregungsausbreitungsrichtung ergibt, die für eine beidseitig erfolgreiche Stimulation charakteristisch ist.

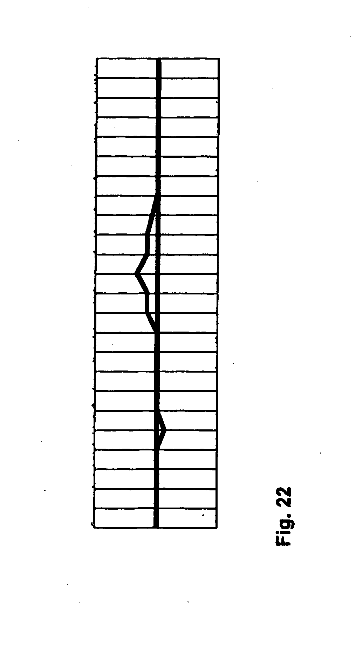

Wie Figur 22 zeigt, kann die auf beschriebene Weise gewonnene Information über rechts- oder linksventrikuläre Capture-Verluste zu einem Bericht zusammengefasst werden, der auf telemetrischen Wege vom implantierbaren Stimulationsgerät, beispielsweise zu einem Homemonitoring Center übertragen werden kann. Figur 18 zeigt das mögliche Aussehen eines solchen Berichtes. Um einen solchen Bericht zu erzeugen, ist die Signalverarbeitungseinheit ausgebildet, tageweise Ereignisse mangelnden rechts- oder linksventrikulären Stimulationserfolges zu zählen und diese Zahlen telemetrisch an ein Homemonitoring Center zu versenden.

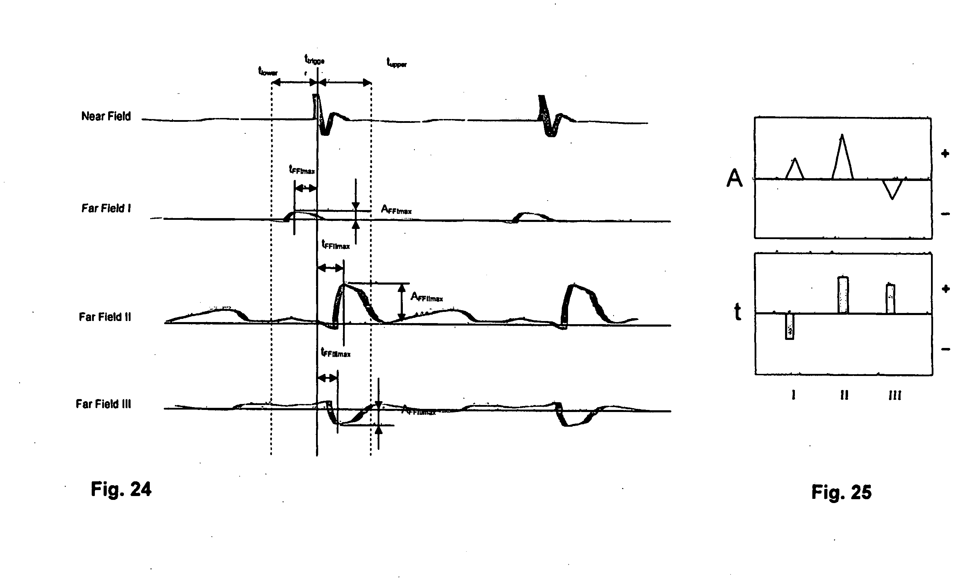

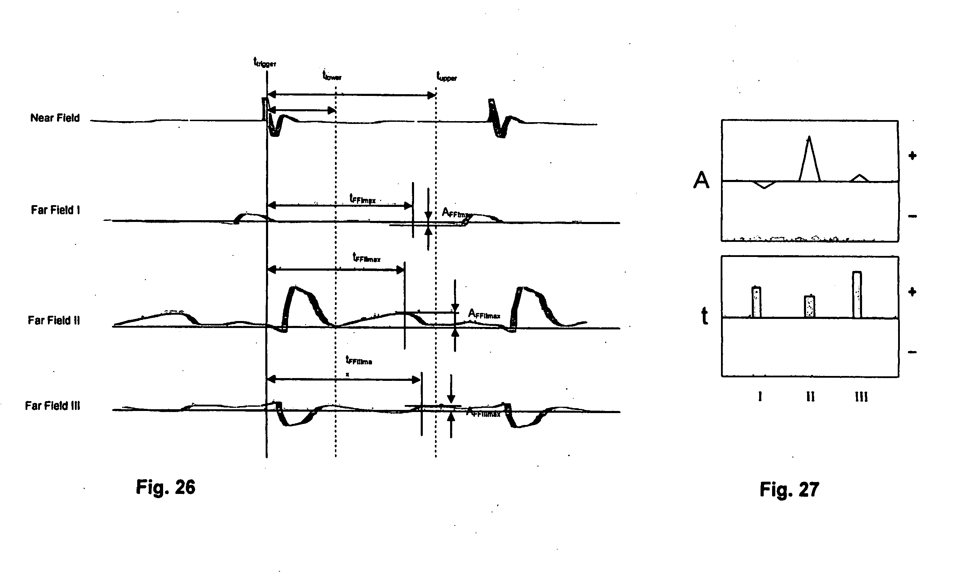

In den Figuren 24 bis 27 ist dargestellt, wie die Eingangssignale von drei Fernfeldkanälen innerhalb eines Zeitfensters ausgewertet werden, welches auf einen Zeitpunkt t_{trigger} bezogen ist, der aus dem Eingangssignal eines Nahfeldkanals abgeleitet wird. In Figur 24 beginnt das Zeitfenster zu einem Zeitpunkt t_{lower}, der vor dem Zeitpunkt t_{trigger} liegt und endet mit einem Zeitpunkt tᵤₚₚₑᵣ, der nach dem Zeitpunkt t_{trigger} liegt. Die Ausführungsvariante gemäß Figur 24 erfordert somit einen Loop-Recorder zur Aufzeichnung der insgesamt vier Eingangssignale. Bei der Ausführungsvariante gemäß Figuren 26 und 27 beginnt das Zeitfenster mit einem Zeitpunkt t_{lower}, der nach dem Zeitpunkt t_{trigger} liegt, so dass für die Ausführungsvariante gemäß Figuren 26 und 27 kein Loop-Recorder erforderlich ist.

Zu beachten ist, dass bei den Ausführungsbeispielen gemäß Figuren 24 bis 27 immer nur die Maximalamplituden (A_{FFmax})der jeweiligen Fernfeldsignale innerhalb des Zeitfensters, also zwischen den Zeitpunkten t_{lower} und tᵤₚₚₑᵣ erfasst werden. Figur 26 zeigt, dass dies nicht notwendigerweise die absoluten Amplitudenmaxima der jeweiligen Fernfeldsignale innerhalb eines jeweiligen Herzzyklusses sind.

Außerdem zeigen die Figuren 24 bis 27, dass neben den Maximalamplitudenwerten A_{FFmax} innerhalb des Zeitfensters jeweils auch die Zeitpunkte (t_{FFmax}) des Auftretens dieser Maximalamplituden erfasst und ausgewertet werden. Diese Zeitpunkte lassen sich - ähnlich den Maximalamplitudenwerten - ebenfalls 3-Tupel als darstellen, der in Figuren 25 und 27 in kompakter Weise graphisch dargestellt ist.

## Patentansprüche

1. Implantierbares Elektrostimulationsgerät, mit wenigstens drei Elektrodenleitungen, welche jeweils mindestens eine Elektrode aufweisen oder mit Anschlüssen zur Verbindung mit wenigstens drei Elektrodenleitungen, welche jeweils mindestens eine Elektrode aufweisen, sowie mit wenigstens drei Eingangskanälen, die mit je wenigstens einer solchen Elektrode oder mit einem Anschluss für eine solche Elektrode verbunden sind, mit der sich wenigstens drei verschiedene mit einer Erregung des Herzgewebes (Myokard) einhergehende elektrische Potentiale in einem Herzen erfassen lassen, wobei jeder der Eingangskanäle einen Sense-Kanal bildet,
**dadurch gekennzeichnet, dass**
das implantierbare Elektrostimulationsgerät eine Signalverarbeitungseinheit aufweist,
- die mit den Eingangskanälen verbunden und ausgebildet ist, den zeitlichen Verlauf der über die drei Sense-Kanäle erfassten Potentiale als drei Eingangssignale in zeitlichem Bezug zu einem periodisch wiederkehrenden Triggersignal auszuwerten, welches jeweils ein Zeitfenster auslöst, und
- die weiterhin ausgebildet ist, für jedes der drei Eingangssignale vorgegebene Signalmerkmale innerhalb des von dem Triggersignal ausgelösten Zeitfensters zu erfassen, zu speichern und mit entsprechenden Signalmerkmalen vorangegangener Zeitfenster und eines jeweils anderen Eingangskanals innerhalb desselben Zeitfensters zu vergleichen.

2. Implantierbares Elektrostimulationsgerät nach Anspruch 1, **dadurch gekennzeichnet, dass** die Signalverarbeitungseinheit ausgebildet ist, ein erfasstes aktuelles Signalmerkmal eines Eingangssignals hinsichtlich des Zeitpunktes des Auftretens eines Formmerkmals in dem Zeitfenster mit dem Zeitpunkt des Auftretens eines entsprechenden Formmerkmals in einem anderen Eingangssignal oder in einem vorangegangenen Zeitfenster zu vergleichen.

3. Implantierbares Elektrostimulationsgerät nach Anspruch 1 oder 2, **dadurch gekennzeichnet, dass** die Signalverarbeitungseinheit ausgebildet ist, den Zeitpunkt des Auftretens eines Amplitudenmaximums in dem Zeitfenster als Signalmerkmal zu erfassen.

4. Implantierbares Elektrostimulationsgerät nach einem der Ansprüche 1 bis 3, **dadurch gekennzeichnet, dass** die Signalverarbeitungseinheit ausgebildet ist, den Zeitpunkt des Auftretens des Signalmerkmals in Bezug auf die Dauer des Zeitfensters zu erfassen.

5. Implantierbares Elektrostimulationsgerät nach einem der Ansprüche 1 bis 4, **dadurch gekennzeichnet, dass** die Signalverarbeitungseinheit ausgebildet ist, das Triggersignal aus einem der Eingangssignale derart abzuleiten, dass das Triggersignal durch ein Signalmerkmal dieses Eingangssignals ausgelöst wird.

6. Implantierbares Elektrostimulationsgerät nach Anspruch 5, **dadurch gekennzeichnet, dass** die Signalverarbeitungseinheit ausgebildet ist, das Triggersignal aus einem Eingangssignal abzuleiten, das ein Nahfeldpotential repräsentiert.

7. Implantierbares Elektrostimulationsgerät nach Anspruch 5 oder 6, **dadurch gekennzeichnet, dass** das implantierbare Elektrostimulationsgerät einen vierten Eingangskanal als vierten Sense-Kanal aufweist, der mit wenigstens einer solchen Elektrode oder mit einem Anschluss für eine solche Elektrode verbunden ist, mit der sich ein Nahfeld eines mit einer Erregung des Herzgewebes (Myokard) einhergehenden elektrischen Potentials in einem Herzen erfassen lässt, und die Signalverarbeitungseinheit ausgebildet ist, das Triggersignal aus dem Eingangssignal des vierten Sense-Kanals abzuleiten.

8. Implantierbares Elektrostimulationsgerät nach Anspruch 6 und 7, **dadurch gekennzeichnet, dass** die ersten drei Sense-Kanäle Fernfeldkanäle sind und mit je einer solchen Elektrode oder mit einem Anschluss für eine solche Elektrode verbunden sind, mit der im Betriebszustand des implantierbaren Elektrostimulationsgerätes jeweils ein Fernfeld eines mit einer Erregung des Herzgewebes (Myokard) einhergehenden elektrischen Potentials zu erfassen ist, und dass der vierte Sense-Kanal eine Nahfeld-Kanal ist, der mit einer solchen Elektrode oder mit einem Anschluss für eine solche Elektrode verbunden ist, mit der im Betriebszustand des implantierbaren Elektrostimulationsgerätes ein Nahfeld eines mit einer Erregung des Herzgewebes (Myokard) einhergehenden elektrischen Potentials zu erfassen ist.

9. Implantierbares Elektrostimulationsgerät nach einem der Ansprüche 6 bis 8, **dadurch gekennzeichnet, dass** ein Nahfeld repräsentierendes Eingangssignal über einen Nahfeld-Kanal zu erfassen ist, an den eine wenigstens bipolare, rechtsventrikuläre Elektrodenleitung angeschlossen oder anzuschließen ist, die eine ventrikuläre Ringelektrode und eine ventrikuläre Tip-Elektrode aufweist, über die das Nahfeld zu erfassen ist.

10. Implantierbares Elektrostimulationsgerät nach einem der Ansprüche 1 bis 9, **dadurch gekennzeichnet, dass** die Signalverarbeitungseinheit einen Loop-Recorder zu Speichern von Eingangssignalabschnitten aufweist und ausgebildet ist, das Erfassen vorgegebener Signalmerkmale in einem durch das Triggersignal ausgelösten Zeitfenster durchzuführen, welches vor dem Auftreten des Triggersignal beginnt.

11. Implantierbares Elektrostimulationsgerät nach einem der Ansprüche 1 bis 10, **dadurch gekennzeichnet, dass** die Signalverarbeitungseinheit über einen Signalschablonenspeicher verfügt und ausgebildet ist, die vorgegebenen Signalmerkmale anhand eines Vergleiches eines jeweiligen Eingangssignals mit einer gespeicherten Signalschablone zu erfassen.

12. Implantierbares Elektrostimulationsgerät nach einem der Ansprüche 1 bis 11, **dadurch gekennzeichnet, dass** wenigstens drei Eingangskanäle Fernfeld-Kanäle sind, die mit je einer solchen Elektrode oder mit einem Anschluss für eine solche Elektrode verbunden sind, mit der im Betriebszustand des implantierbaren Elektrostimulationsgerätes jeweils ein Fernfeld eines mit einer Erregung des Herzgewebes (Myokard) einhergehenden elektrischen Potentials zu erfassen ist, so dass an den Eingangskanälen ein ein jeweiliges Fernfeld repräsentierendes Eingangssignal anliegt, und dass die Signalverarbeitungseinheit ausgebildet ist, drei voneinander verschiedene Differenzsignale zu bilden, die sich aus der Differenz zweier an je einem Eingangskanal anliegenden Eingangssignalen ergeben, und für einen jeweiligen Herzzyklus für jedes der Differenzsignale einen jeweiligen Maximumamplitudenwert zu bilden, der die maximale Auslenkung des jeweiligen Differenzsignals innerhalb des jeweiligen Herzzyklusses repräsentiert.

13. Implantierbares Elektrostimulationsgerät nach Anspruch 12, **dadurch gekennzeichnet, dass** das Elektrostimulationsgerät als implantierbarer Kardioverter/Defibrillator ausgebildet ist, und dass von den drei Fernfeld-Kanälen ein erster mit einer vom (aktiven) Gehäuse des implantierbaren Kardioveter/Defibrillator gebildeten Elektrode und einem Anschluss für eine proximale Defibrillationselektrode (atriale Schockelektrode), ein zweiter Eingangskanal mit der vom Gehäuse des implantierbaren Kardioverter/Defibrillator gebildeten Elektrode einem Anschluss für eine distale Defibrillationselektrode (ventrikuläre Schockelektrode) und ein dritter Eingangskanal mit dem Anschluss für eine distale Defibrillationselektrode und dem Anschluss für eine proximale Defibrillationselektrode verbunden ist.

14. Implantierbares Elektrostimulationsgerät nach Anspruch 12 oder 13, **dadurch gekennzeichnet, dass** die Signalverarbeitungseinheit ausgebildet ist, über die drei Fernfeld-Kanäle erfasste Differenzsignale jeweils hinsichtlich Betrag und Vorzeichen ihrer Maximumamplitudenwert im Verhältnis zueinander auszuwerten.

15. Implantierbares Elektrostimulationsgerät nach einem der Ansprüche 12 bis 14, **dadurch gekennzeichnet, dass** die Signalverarbeitungseinheit ausgebildet ist, aus über je zwei Fernfeldkanäle empfangenen Differenzsignalen je ein Elektrokardiogramm abzuleiten.

16. Implantierbares Elektrostimulationsgerät nach Anspruch 15, **dadurch gekennzeichnet, dass** die Signalverarbeitungseinheit ausgebildet ist, drei Elektrokardiogramme aus denjenigen Differenzsignalen abzuleiten, die von drei unterschiedlichen, jeweils paarweise zusammenwirkenden Eingangskanälen stammen.

17. Implantierbares Elektrostimulationsgerät nach Anspruch 16, **dadurch gekennzeichnet, dass** die Signalverarbeitungseinheit ausgebildet ist, für jedes der Elektrokardiogramme die jeweilige maximale Auslenkung (Amplitude) im Bereich des QRS-Komplexes als Signalmerkmal zu bestimmen.

18. Implantierbares Elektrostimulationsgerät nach einem der Ansprüche 12 bis 17, **dadurch gekennzeichnet, dass** das Elektrostimulationsgerät über einen Speicher verfügt, in dem für jedes der Differenzsignale oder der Elektrokardiogramme ein Winkelwert gespeichert ist, wobei die Signalverarbeitungseinheit mit dem Speicher verbunden und ausgebildet ist, die jeweilige Maximumamplitude des jeweiligen Differenzsignals oder die Amplitude des jeweiligen Elektrokardiogramms zusammen mit dem zugehörigen Winkelwert auszuwerten.

19. Implantierbares Elektrostimulationsgerät nach Anspruch 18, **dadurch gekennzeichnet, dass** die Signalverarbeitungseinheit ausgebildet ist, die Maximumamplituden der Differenzsignale oder die maximalen Auslenkungen der Elektrokardiogramme als Vektorkomponenten eines Summenvektors auszuwerten und die Richtung (und Orientierung) des Summenvektors zu bestimmen und auszuwerten.

20. Implantierbares Elektrostimulationsgerät nach Anspruch 19, **dadurch gekennzeichnet, dass** die Signalverarbeitungseinheit ausgebildet ist, den Summenvektor als Einthovenschen Summenvektor aus den maximalen Auslenkungen dreier unterschiedlicher Elektrokardiogramme zum Zeitpunkt einer R-Zacke eines jeweiligen Herzzyklusses abzuleiten.

21. Implantierbares Elektrostimulationsgerät nach einem der Ansprüche 18 bis 20, **dadurch gekennzeichnet, dass** die Signalverarbeitungseinheit ausgebildet ist, die Richtung des Summenvektors für jeden Herzzyklus neu zu bestimmen.

22. Implantierbares Elektrostimulationsgerät nach Anspruch 21, **dadurch gekennzeichnet, dass** die Signalverarbeitungseinheit mit einem Speicher für wenigstens die Richtung des Summenvektors für wenigstens einen vorangegangenen Herzzyklus zusammenwirkt und ausgebildet ist, eine Änderung der Richtung des Summenvektors über ein vorgegebenes Mindestmaß hinaus zu detektieren.

23. Implantierbares Elektrostimulationsgerät nach Anspruch 22, **dadurch gekennzeichnet, dass** die Signalverarbeitungseinheit ausgebildet ist, eine Änderung der Richtung des Summenvektors für mehr als einen Herzzyklus zu detektieren.

24. Implantierbares Elektrostimulationsgerät nach Anspruch 6 und 7 sowie einem der übrigen Ansprüche 2 bis 4 und 8 bis 23, **dadurch gekennzeichnet, dass** die Signalverarbeitungseinheit ausgebildet ist, das das Nahfeld kennzeichnende Eingangssignal hinsichtlich jeweils eine Kammerkontraktion kennzeichnender Signalspitzen auszuwerten.

25. Implantierbares Elektrostimulationsgerät nach Anspruch 24, **dadurch gekennzeichnet, dass** die Signalverarbeitungseinheit ausgebildet ist, den zeitlichen Abstand der Signalspitzen hinsichtlich einer Herzrate oder eines RR-Intervalls auszuwerten.

26. Implantierbares Elektrostimulationsgerät nach Anspruch 24 oder 25, **dadurch gekennzeichnet, dass** die Signalverarbeitungseinheit ausgebildet ist, aus den Signalspitzen eine Markersignal abzuleiten.

27. Implantierbares Elektrostimulationsgerät nach Anspruch 26, **dadurch gekennzeichnet, dass** die Signalverarbeitungseinheit ausgebildet ist, die Amplitude (Auslenkung) des Differenzsignals oder des Elektrokardiogramms für jeden Herzzyklus zum Zeitpunkt eines jeweiligen Markersignals zu bestimmen und die so gewonnenen Amplituden als Beträge der Komponenten eines Summenvektors zur Bestimmung der Richtung des Summenvektors auszuwerten.

28. Implantierbares Elektrostimulationsgerät nach einem der Ansprüche 1 bis 27,
**dadurch gekennzeichnet, dass** das Elektrostimulationsgerät eine Tachykardieklassifikationseinheit aufweist, die mit der Signalverarbeitung verbunden und ausgebildet ist, eine ventrikuläre Tachykardie durch Auswerten der RR-Intervalle oder der RR-Rate sowie von Betrag und Vorzeichen der Maximumamplitudenwerte der Differenzsignale oder von Betrag und Vorzeichen der Amplituden der Elektrokardiogramme zu klassifizieren.

29. Implantierbares Elektrostimulationsgerät nach Anspruch 25 und einem der Ansprüche 19 bis 23, **dadurch gekennzeichnet, dass** das Elektrostimulationsgerät eine Tachykardieklassifikationseinheit aufweist, die mit der Signalverarbeitung verbunden und ausgebildet ist, eine ventrikuläre Tachykardie durch Auswerten der Richtung des Summenvektors und der RR-Intervalle oder der RR-Rate zu klassifizieren.

30. Implantierbares Elektrostimulationsgerät nach einem der Ansprüche 1 bis 29, **dadurch gekennzeichnet, dass** das Elektrostimulationsgerät eine Stimulationserfolgserfassungseinheit aufweist, die mit der Signalverarbeitung verbunden und ausgebildet ist, eine stimulierte Kontraktion einer Herzkammer anhand von Betrag und Vorzeichen der Maximumamplitudenwerte der Differenzsignale oder von Betrag und Vorzeichen der Amplituden der Elektrokardiogramme zu erfassen und einer Herzkammer zuzuordnen.

31. Implantierbares Elektrostimulationsgerät nach einem der Ansprüche 19 bis 23, **dadurch gekennzeichnet, dass** das Elektrostimulationsgerät eine Stimulationserfolgserfassungseinheit aufweist, die mit der Signalverarbeitung verbunden und ausgebildet ist, eine stimulierte Kontraktion einer Herzkammer durch Auswerten der Richtung des Summenvektors zu erfassen und einer Herzkammer zuzuordnen.

## Claims

1. An implantable electrostimulation device, having at least three electrode lines, each of which has at least one electrode, or having terminals for connection to at least three electrode lines, each of which has at least one electrode, and having at least three input channels, each of which is connected to at least one such electrode or to a terminal for such an electrode, with which at least three different electric potentials associated with stimulation of the cardiac tissue (myocardium) can be detected in the heart, each of these input channels forming a sense channel,
**characterized in that** the implantable electro-stimulation device has a signal processing unit,
- which is connected to the input channels and is designed to evaluate the chronological course of the potentials detected via the three sense channels as three input signals in chronological reference to a periodically recurring trigger signal, each of which triggers a time window, and
- which is also designed to detect and store signal features predefined for each of the three input signals within the time window triggered by the trigger signal and compare them with corresponding signal features of preceding time windows and another respective input channel within the same time window.

2. The implantable electrostimulation device according to claim 1, **characterized in that** the signal processing unit is designed to compare a detected current signal feature of an input signal with regard to the point in time of occurrence of a shape feature in the time window with the point in time of occurrence of a corresponding shape feature in another input signal or in a preceding time window.

3. The implantable electrostimulation device according to claim 1 or 2, **characterized in that** the signal processing unit is designed to detect the point time of occurrence of a maximum amplitude in the time window as a signal feature.

4. The implantable electrostimulation device according to any one of claims 1 to 3, **characterized in that** the signal processing unit is designed to detect the point in time of occurrence of the signal feature with respect to the duration of the time window.

5. The implantable electrostimulation device according to any one of claims 1 to 4, **characterized in that** the signal processing unit is designed to derive the trigger signal from one of the input signals, such that the trigger signal is triggered by a signal feature of this input signal.

6. The implantable electrostimulation device according to claim 5, **characterized in that** the signal processing unit is designed to derive the trigger signal from an input signal, which represents a near-field potential.

7. The implantable electrostimulation device according to claim 5 or 6, **characterized in that** the implantable electrostimulation device has a fourth input channel as a fourth sense channel, which is connected to at least one such electrode or to a terminal for such an electrode, with which a near field of an electric potential associated with stimulation of the cardiac tissue (myocardium) in the heart can be detected, and the signal processing unit is designed to derive the trigger signal from the input signal of the fourth sense channel.

8. The implantable electrostimulation device according to claims 6 and 7, **characterized in that** the first three sense channels are far-field channels and each is connected to such an electrode or to a terminal for such an electrode, with which a far field of an electric potential associated with stimulation of the cardiac tissue (myocardium) can be detected in the operating mode of the implantable electro-stimulation device, and the fourth sense channel is a near-field channel, which is connected to such an electrode or to a terminal for such an electrode with which a near field of an electric potential associated with stimulation of the cardiac tissue (myocardium) is to be detected in the operating mode of the implantable electrostimulation device.

9. The implantable electrostimulation device according to any one of claims 6 to 8, **characterized in that** an input signal representing a near field is to be detected via a near-field channel, to which an at least bipolar right-ventricular electrode line is connected or is to be connected, having a ventricular ring electrode and a ventricular tip electrode, by means of which the near field is to be detected.

10. The implantable electrostimulation device according to any one of claims 1 to 9, **characterized in that** the signal processing unit has a loop recorder for storing input signal sections and is designed to perform the detection of predefined signal features in a time window triggered by the trigger signal, beginning before the occurrence of the trigger signal.

11. The implantable electrostimulation device according to any one of claims 1 to 10, **characterized in that** the signal processing unit has a signal template memory and is designed to detect the predetermined signal features on the basis of a comparison of a respective input signal with a stored signal template.

12. The implantable electrostimulation device according to any one of claims 1 to 11, **characterized in that** at least three input channels are far-field channels, each of which is connected to such an electrode or to a terminal for such an electrode, with which a far field of an electric potential associated with stimulation of the cardiac tissue (myocardium) is to be detected in the operating mode of the implantable electrostimulation device, so that an input signal representing a respective far field is applied to the input channels, and the signal processing unit is designed to form three different differential signals, which are derived from the difference between two input signals applied to one input channel each, and to form a respective maximum amplitude value for a respective cardiac cycle for each of the differential signals, representing the maximum deflection of the respective differential signal within the respective cardiac cycle.

13. The implantable electrostimulation device according to claim 12, **characterized in that** the electrostimulation device is designed as an implantable cardioverter/defibrillator, and of the three far-field channels, a first input channel with an electrode formed by the active housing of the implantable cardioverter/defibrillator and a terminal for a proximal defibrillation electrode (atrial shock electrode), a second input channel with the electrode formed by the housing of the implantable cardioverter/defibrillator is connected to a terminal for a distal defibrillation electrode (ventricular shock electrode), and a third input channel is connected to the terminal for a distal defibrillation electrode and the terminal for a proximal defibrillation electrode.

14. The implantable electrostimulation device according to claim 12 or 13, **characterized in that** the signal processing unit is designed to evaluate differential signals detected via the three far-field channels with regard to the value and sign of their maximum amplitude value in relation to one another.

15. The implantable electrostimulation device according to any one of claims 12 to 14, **characterized in that** the signal processing unit is designed to derive an electrocardiogram from differential signals each received via two far-field channels.

16. The implantable electrostimulation device according to claim 15, **characterized in that** the signal processing unit is designed to derive three electrocardiograms from the differential signals which originate from three different input channels cooperating in pairs, respectively.

17. The implantable electrostimulation device according to claim 16, **characterized in that** the signal processing unit is designed to determine the respective maximum deflection (amplitude) in the range of the QRS complex as a signal feature for each of the electrocardiograms.

18. The implantable electrostimulation device according to any one of claims 12 to 17, **characterized in that** the electrostimulation device has a memory in which an angle value is stored for each of the differential signals or the electrocardiograms, the signal processing unit being connected to the memory and designed to evaluate the maximum amplitude of the respective differential signal or the amplitude of the respective electrocardiogram together with the respective angle value.

19. The implantable electrostimulation device according to claim 18, **characterized in that** the signal processing unit is designed to evaluate the maximum amplitudes of the differential signals or the maximum deflections of the electrocardiograms as vector components of a sum vector and to determine and evaluate the direction (and orientation) of the sum vector.

20. The implantable electrostimulation device according to claim 19, **characterized in that** the signal processing unit is designed to derive the sum vector as the Einthoven sum vector from the maximum deflections of three different electrocardiograms at the point in time of an R wave of a respective cardiac cycle.

21. The implantable electrostimulation device according to any one of claims 18 to 20, **characterized in that** the signal processing unit is designed to determine anew the direction of the sum vector for each cardiac cycle.

22. The implantable electrostimulation device according to claim 21, **characterized in that** the signal processing unit cooperates with a memory for at least the direction of the sum vector for at least one preceding cardiac cycle and is designed to detect a change in the direction of the sum vector beyond a predetermined minimum extent.

23. The implantable electrostimulation device according to claim 22, **characterized in that** the signal processing unit is designed to detect a change in the direction of the sum vector for more than one cardiac cycle.

24. The implantable electrostimulation device according to claims 6 and 7 as well as any one of the other claims 2 to 4 and 8 to 23, **characterized in that** the signal processing unit is designed to evaluate the input signal characterizing the near field with regard to signal peaks characterizing a chamber contraction, respectively.

25. The implantable electrostimulation device according to claim 24, **characterized in that** the signal processing unit is designed to evaluate the interval in time between the signal peaks with regard to a heart rate or an RR interval.

26. The implantable electrostimulation device according to claim 24 or 25, **characterized in that** the signal processing unit is designed to derive a marker signal from the signal peaks.

27. The implantable electrostimulation device according to claim 26, **characterized in that** the signal processing unit is designed to determine the amplitude (deflection) of the differential signal or of the electrocardiogram for each cardiac cycle at the time of a respective marker signal and to evaluate the amplitudes thereby obtained as the values of components of a sum vector for determining the direction of the sum vector.

28. The implantable electrostimulation device according to any one of claims 1 to 27, **characterized in that** the electrostimulation device is a tachycardia classification unit, which is connected to the signal processing unit and is designed to classify a ventricular tachycardia by evaluating the RR intervals or the RR rate and the value and sign of the maximum amplitude values of the differential signals or the value and sign of the amplitudes of the electrocardiograms.

29. The implantable electrostimulation device according to claim 25 and any one of claims 19 to 23, **characterized in that** the electrostimulation device has a tachycardia classification unit, which is connected to the signal processing unit and is designed to classify a ventricular tachycardia by evaluating the direction of the sum vector and the RR intervals or the RR rate.

30. The implantable electrostimulation device according to any one of claims 1 to 29, **characterized in that** the electrostimulation device has a capture control detection unit, which is connected to the signal processing unit and is designed to detect a stimulated contraction of a cardiac chamber on the basis of the value and sign of the maximum amplitude values of the differential signals or the value and sign of the amplitudes of the electrocardiograms and to assign it to a cardiac chamber.

31. The implantable electrostimulation device according to any one of claims 19 to 23, **characterized in that** the electrostimulation device has a capture detection unit, which is connected to the signal processing unit and is designed to detect a stimulated contraction of a cardiac chamber by evaluating the direction of the sum vector and to assign it to a cardiac chamber.

## Revendications

1. Appareil de stimulation électrique implantable, comportant au moins trois conducteurs à électrode présentant chacun au moins une électrode ou comportant des raccordements pour le raccorder à au moins trois conducteurs à électrode présentant chacun au moins une électrode, ainsi qu'au moins trois canaux d'entrée qui sont reliés chacun à au moins une telle électrode ou à un raccordement pour une telle électrode qui permet de détecter au moins trois potentiels électriques différents allant de pair avec une excitation du tissu cardiaque (myocarde), chacun des canaux d'entrée formant un canal de détection,
**caractérisé en ce que**
l'appareil de stimulation électrique implantable présente une unité de traitement de signaux,
- qui est reliée aux canaux d'entrée et est conçue pour évaluer l'évolution chronologique des potentiels détectés par les trois canaux de détection sous forme de trois signaux d'entrée en relation chronologique avec un signal de déclenchement revenant périodiquement et déclenchant respectivement une fenêtre temporelle et
- qui est en outre conçue pour détecter pour chacun des trois signaux d'entrée des caractéristiques de signal prédéfinies dans la fenêtre temporelle déclenchée par le signal de déclenchement, pour les enregistrer et les comparer à des caractéristiques de signal correspondantes de fenêtres temporelles précédentes et d'un autre canal respectif dans la même fenêtre temporelle.

2. Appareil de stimulation électrique implantable selon la revendication 1, **caractérisé en ce que** l'unité de traitement de signal est conçue pour comparer une caractéristique de signal actuelle détectée d'un signal d'entrée par rapport au moment de la survenance d'une caractéristique de forme dans la fenêtre temporelle avec le moment de la survenance d'une caractéristique de forme correspondante dans un autre signal d'entrée ou dans une fenêtre temporelle précédente.

3. Appareil de stimulation électrique implantable selon la revendication 1 ou 2, **caractérisé en ce que** l'unité de traitement de signal est conçue pour détecter le moment de la survenance d'un maximum d'amplitude dans la fenêtre temporelle sous forme de caractéristique de signal.

4. Appareil de stimulation électrique implantable selon une des revendications 1 à 3, **caractérisé en ce que** l'unité de traitement de signal est conçue pour détecter le moment de la survenance de la caractéristique de signal par rapport à la durée de la fenêtre temporelle.

5. Appareil de stimulation électrique implantable selon une des revendications 1 à 4, **caractérisé en ce que** l'unité de traitement de signal est conçue pour dériver le signal de déclenchement d'un des signaux d'entrée de manière à ce que le signal de déclenchement soit déclenché par une caractéristique de signal de ce signal d'entrée.

6. Appareil de stimulation électrique implantable selon la revendication 5, **caractérisé en ce que** l'unité de traitement de signal est conçue pour dériver le signal de déclenchement d'un signal d'entrée qui représente un potentiel de champ proche.

7. Appareil de stimulation électrique implantable selon la revendication 5 ou 6, **caractérisé en ce que** l'appareil de stimulation électrique implantable présente un quatrième canal d'entrée faisant office de quatrième canal de détection et qui est relié à au moins une telle électrode ou à un raccordement pour une telle électrode qui permet d'enregistrer un champ proche d'un potentiel électrique allant de pair avec une excitation du tissu cardiaque (myocarde) dans un coeur et que l'unité de traitement de signal est conçue pour dériver le signal de déclenchement du signal d'entrée du quatrième canal de détection.

8. Appareil de stimulation électrique implantable selon les revendications 6 et 7, **caractérisé en ce que** les trois premiers canaux de détection sont des canaux de champ proche et sont reliés chacun à une telle électrode ou à un raccordement pour une telle électrode qui permet, en situation de fonctionnement de l'appareil de stimulation électrique implantable, de détecter respectivement un champ lointain d'un potentiel électrique allant de pair avec une excitation du tissu cardiaque (myocarde) et que le quatrième canal de détection est un canal de champ proche qui est relié à une telle électrode ou à un raccordement pour une telle électrode qui permet, en situation de fonctionnement de l'appareil de stimulation électrique implantable, de détecter un champ proche d'un potentiel électrique allant de pair avec une excitation du tissu cardiaque (myocarde).

9. Appareil de stimulation électrique implantable selon une des revendications 6 à 8, **caractérisé en ce qu'**un signal d'entrée représentant un champ proche se détecte par un canal de champ proche auquel est raccordé ou doit être raccordé un conducteur à électrode ventriculaire droit au moins bipolaire qui présente une électrode ventriculaire annulaire et une électrode ventriculaire à pointe qui permet de détecter le champ proche.

10. Appareil de stimulation électrique implantable selon une des revendications 1 à 9, **caractérisé en ce que** l'unité de traitement de signal présente une mémoire de boucle pour l'enregistrement de segments de signal d'entrée et est conçue pour réaliser la détection des caractéristiques de signal prédéfinies dans une fenêtre temporelle déclenchée par le signal de déclenchement, laquelle détection débute avant la survenance du signal de déclenchement.

11. Appareil de stimulation électrique implantable selon une des revendications 1 à 10, **caractérisé en ce que** l'unité de traitement de signal dispose d'une mémoire de modèle de signal et est conçue pour détecter les caractéristiques de signal prédéfinies en partant d'une comparaison d'un signal d'entrée respectif avec un modèle de signal enregistré.

12. Appareil de stimulation électrique implantable selon une des revendications 1 à 11, **caractérisé en ce qu'**au moins trois canaux d'entrée sont des canaux de champ lointain qui sont reliés chacun à une telle électrode ou à un raccordement pour une telle électrode qui permet, en situation de fonctionnement de l'appareil de stimulation électrique implantable, de détecter respectivement un champ lointain d'un potentiel électrique allant de pair avec une excitation du tissu cardiaque (myocarde), de sorte que, au niveau des canaux d'entrée, est appliqué un signal d'entrée représentant un champ lointain respectif, et que l'unité de traitement de signal est conçue pour former trois signaux différentiels différents les uns des autres qui résultent de la différence entre deux signaux appliqués chacun au niveau d'un canal d'entrée et pour établir, pour un cycle cardiaque respectif, pour chacun des signaux différentiels, une valeur d'amplitude maximale respective qui représente la déviation maximale du signal différentiel respectif dans le cycle cardiaque respectif.

13. Appareil de stimulation électrique implantable selon la revendication 12, **caractérisé en ce que** l'appareil de stimulation électrique se présente sous la forme d'un défibrillateur implantable et que, parmi les trois canaux ce champ lointain, un premier est relié à une électrode formée par le boîtier (actif) du défibrillateur implantable et à un raccordement pour une électrode proximale de défibrillation (électrode atriale de choc), un deuxième canal d'entrée à l'électrode formée par le boîtier du défibrillateur implantable et à un raccordement pour une électrode distale de défibrillation (électrode ventriculaire de choc) et un troisième canal d'entrée au raccordement pour une électrode distale de défibrillation et au raccordement pour une électrode proximale de défibrillation.

14. Appareil de stimulation électrique implantable selon la revendication 12 ou 13, **caractérisé en ce que** l'unité de traitement de signal est conçue pour évaluer les uns par rapport aux autres les signaux différentiels détectés par les trois canaux de champ lointain respectivement en ce qui concerne le montant et le signe de leur valeur d'amplitude maximale.

15. Appareil de stimulation électrique implantable selon une des revendications 12 à 14, **caractérisé en ce que** l'unité de traitement de signal est conçue pour dériver chaque fois un électrocardiogramme des signaux différentiels reçus chaque fois par deux canaux de champ lointain.

16. Appareil de stimulation électrique implantable selon la revendication 15, **caractérisé en ce que** l'unité de traitement de signal est conçue pour dériver trois électrocardiogrammes des signaux différentiels provenant de trois canaux d'entrée différents et coopérant respectivement par paires.

17. Appareil de stimulation électrique implantable selon la revendication 16, **caractérisé en ce que** l'unité de traitement de signal est conçue pour définir, pour chacun des électrocardiogrammes, la déviation maximale respective (amplitude) au niveau du complexe QRS sous forme de caractéristique de signal.

18. Appareil de stimulation électrique implantable selon une des revendications 12 à 17, **caractérisé en ce que** l'appareil de stimulation électrique dispose d'une mémoire dans laquelle une valeur d'angle est enregistrée pour chacun des signaux différentiels ou des électrocardiogrammes, l'unité de traitement de signal étant reliée à la mémoire et conçue pour évaluer l'amplitude maximale respective du signal différentiel respectif ou l'amplitude de l'électrocardiogramme respectif en même temps que la valeur d'angle respective.

19. Appareil de stimulation électrique implantable selon la revendication 18, **caractérisé en ce que** l'unité de traitement de signal est conçue pour définir et évaluer les amplitudes maximales des signaux différentiels ou les déviations maximales des électrocardiogrammes sous forme de composants vectoriels d'un vecteur de somme et le sens (et l'orientation) du vecteur de somme.

20. Appareil de stimulation électrique implantable selon la revendication 19, **caractérisé en ce que** l'unité de traitement de signal est conçue pour dériver le vecteur de somme en tant que vecteur de somme d'Einthoven des déviations maximales de trois électrocardiogrammes différents au moment d'une onde R d'un cycle cardiaque respectif.

21. Appareil de stimulation électrique implantable selon une des revendications 18 à 20, **caractérisé en ce que** l'unité de traitement de signal est conçue pour redéfinir le sens du vecteur de somme pour chaque cycle cardiaque.

22. Appareil de stimulation électrique implantable selon la revendication 21, **caractérisé en ce que** l'unité de traitement de signal coopère avec une mémoire pour au moins le sens du vecteur de somme au moins pour un cycle cardiaque précédent et est conçue pour détecter une modification du sens du vecteur de somme via une valeur minimale prédéfinie.

23. Appareil de stimulation électrique implantable selon la revendication 22, **caractérisé en ce que** l'unité de traitement de signal est conçue pour détecter une modification du sens du vecteur de somme pour plus d'un cycle cardiaque.

24. Appareil de stimulation électrique implantable selon les revendications 6 et 7 et une des autres revendications 2 à 4 et 8 à 23, **caractérisé en ce que** l'unité de traitement de signal est conçue pour évaluer le signal d'entrée caractérisant le champ proche en ce qui concerne des pointes de signal caractérisant respectivement une contraction de chambre.

25. Appareil de stimulation électrique implantable selon la revendication 24, **caractérisé en ce que** l'unité de traitement de signal est conçue pour évaluer l'écart chronologique entre les pointes de signal en ce qui concerne une fréquence cardiaque ou un intervalle RR.

26. Appareil de stimulation électrique implantable selon la revendication 24 ou 25, **caractérisé en ce que** l'unité de traitement de signal est conçue pour dériver un signal marqueur des pointes de signal.

27. Appareil de stimulation électrique implantable selon la revendication 26, **caractérisé en ce que** l'unité de traitement de signal est conçue pour définir l'amplitude (la déviation) du signal différentiel ou de l'électrocardiogramme pour chaque cycle cardiaque au moment d'un signal marqueur respectif et pour évaluer les amplitudes ainsi obtenues sous forme de montants des composants d'un vecteur de somme pour définir le sens du vecteur de somme.

28. Appareil de stimulation électrique implantable selon une des revendications 1 à 27, **caractérisé en ce que** l'appareil de stimulation électrique présente une unité de classification de tachycardie qui est reliée à l'unité de traitement de signal et est conçue pour classifier une tachycardie ventriculaire par évaluation des intervalles RR ou de la fréquence RR et du montant et du signe des valeurs d'amplitude maximales des signaux différentiels ou du montant et du signe des amplitudes des électrocardiogrammes.

29. Appareil de stimulation électrique implantable selon la revendication 25 et une des revendications 19 à 23, **caractérisé en ce que** l'appareil de stimulation électrique présente une unité de classification de tachycardie qui est reliée à l'unité de traitement de signal et est conçue pour classifier une tachycardie ventriculaire par évaluation du sens du vecteur de somme et des intervalles RR ou de la fréquence RR.

30. Appareil de stimulation électrique implantable selon une des revendications 1 à 29, **caractérisé en ce que** l'appareil de stimulation électrique présente une unité de détection de stimulation qui est reliée à l'unité de traitement de signal et est conçue pour détecter une contraction stimulée d'une chambre cardiaque en partant du montant et du signe des valeurs d'amplitude minimale des signaux différentiels ou du montant et du signe des amplitudes des électrocardiogrammes et les associer à une chambre cardiaque.

31. Appareil de stimulation électrique implantable selon une des revendications 19 à 23, **caractérisé en ce que** l'appareil de stimulation électrique présente une unité de détection de stimulation qui est reliée à l'unité de traitement de signal et est conçue pour détecter une stimulation stimulée d'une chambre cardiaque par évaluation du sens du vecteur de somme et l'associer à une chambre cardiaque.
